# EUROPEAN PATENT APPLICATION

(11) **EP 4 306 154 A2**
(43) Date of publication of application: **17.01.2024**
(21) Application number: 23213304.1
(22) Date of filing: 23.11.2017
(51) Int. Cl.: A61M 13/00

(54) **HIGH FLOW LUER CONNECTOR**

(30) Priority: 23.11.2016 US 201662426052 P; 29.09.2017 US 201762565859 P
(62) Divisional of application: 17873263.2
(71) Applicant: Fisher & Paykel Healthcare Limited, Auckland, 2013 (NZ)
(72) Inventor: BOYES, Richard John, 2013 Auckland (NZ); LAUS, Charlotte Grace, 2013 Auckland (NZ); FISCHER, Christian Francis, 2013 Auckland (NZ); VERDOOLD, Vincent, 2013 Auckland (NZ); IP, Bernard Tsz Lun, 2013 Auckland (NZ); CHAN, Jessica Kristen, 2013 Auckland (NZ); GHALIB, Ali Ghalib Abdul Rahman, 2013 Auckland (NZ)
(74) Representative: Treeby, Philip David William

(57) **Abstract**

In one embodiment, a Luer lock connector for use in an insufflation system is described. The Luer lock connector includes: a body comprising a first end, a second end and an interior region; the interior region defining a gases flow passageway allowing insufflation gases to flow through the body from the first end to the second end; and the body being configured to be coupled to a tubing arrangement at the first end and to a patient interface at the second end; wherein, the second end is configured to be coupled to a patient interface fitting of the patient interface, the second end being further configured to seal around an outer surface of the patient interface fitting when the Luer lock connector is coupled to the patient interface; and wherein, the second end forms a seal with the outer surface of the patient interface fitting when the second end and the patient interface fitting are coupled.

## Description

### BACKGROUND

### 1. Field of the Invention

The present invention generally relates to connectors for medical devices. More specifically, the present invention relates to Luer lock connectors for use in insufflation systems.

### 2. Description of Related Art

Insufflation gases can be used in surgery for a variety of purposes. In open surgery, gas can be insufflated into a body cavity for de-airing, as in cardiac surgery. In laparoscopic surgery, the abdominal wall can be distended using gas to provide room for instrument insertion and tissue dissection.

During these surgical procedures, it may be desirable to infuse insufflation gas into the cavity under controlled operating parameters such as a particular flow rate, pressure, etc. However, controlling the flow rate or obtaining a high flow rate may be difficult because of several limitations. These limitations include, inter alia, constraints placed upon the insufflation equipment by common industry practice, efficacy requirements, and the equipment associated with these surgical procedures such as Luer connectors.

Standardized Luer connectors provide connections for the transfer of fluids or gases between two devices or objects such as veress needles, trocars, syringes, or gas/fluid delivery systems. Although there are many Luer connector sizes available, Luer connections typically operate in the same way, and conform to International Organization of Standard ("ISO") standards 594-1 or 594-2.

Traditional Luer connectors include a matched set of male and female fittings, each having conical or tapered surfaces that, when the fittings are connected, provide leak-proof connections between tubes. Conical fittings may include slip and/or lock fittings. "Slip fittings" were developed for use with liquids where a friction fit was sufficient to provide both sealing and retention functions. "Lock fittings" were developed for use with pressurized gases for which a proper retention mechanism was needed although the slip fitting was traditionally retained to provide the seal.

Luer connectors provide leak-free and secure connections between medical devices. However, the particular configuration of such connectors which include conical or tapered surfaces traditionally narrows the cross-sectional area of the gas flow path and therefore results in an increased resistance to flow. In pressure-regulated systems, the pressure drop across the system is fixed, so an increase in resistance to flow results in a decrease in flow rate. Therefore, it would be desirable to have a Luer connector providing a leak-free and secure connection with less resistance to flow thereby increasing the overall performance of the insufflation system.

### SUMMARY

It is an object of the present invention to provide which at least goes some way towards overcoming the above disadvantages or which will at least provide the public with a useful choice.

In a first aspect, the invention consists in a Luer lock connector for use in an insufflation system, the Luer lock connector including: a body comprising a first end, a second end and an interior region; the interior region defining a gases flow passageway allowing insufflation gases to flow through the body from the first end to the second end; and the body being configured to be coupled to a tubing arrangement at the first end and to a patient interface at the second end; wherein, the second end is configured to be coupled to a patient interface fitting of the patient interface, the second end being further configured to lock and seal around an outer surface of the patient interface fitting when the Luer lock connector is coupled to the patient interface.

In an embodiment, the second end is configured to lock around the outer surface of the patient interface fitting when the patient interface engages with the Luer lock connector.

In another embodiment, the second end of the Luer lock connector is configured to be rotatably engaged with the patient interface fitting.

In a further embodiment, the second end comprises a semi-rigid material and threads on an inner surface. The threads may be configured to be coupled to complementary tabs arranged on the outer surface of the patient interface fitting.

In an embodiment, the second end conforms around the outer surface of the patient interface fitting to form a seal when the second end and the patient interface fitting are coupled.

In another embodiment, the patient interface fitting is configured to be inserted into the second end of the Luer lock connector.

In a further embodiment, the second end comprises a flexible material and one or more ridges on an inner surface.

In an embodiment, the second end is configured to be more flexible or softer than the first end.

In a further embodiment, the one or more ridges are configured to be coupled to a mating structure arranged on the outer surface of the patient interface fitting. The one or more ridges and the mating structure may be configured to form a seal when the second end and the patient interface fitting are coupled.

In an embodiment, the second end further comprises a distal end having an inner diameter that is less than an outer diameter of the patient interface fitting, and wherein the distal end presses onto the outer surface of the patient interface fitting to form a seal when the second end and the patient interface fitting are coupled.

In another embodiment, the second end further comprises a hollow portion adapted to receive a pressurized gas in use so as to strengthen the seal when the second end and the patient interface fitting are coupled.

In a further embodiment, the second end comprises a first portion made of a rigid material and a second portion made of a flexible material. The second portion may be overmoulded over the first portion and the first portion comprises one or more gaps allowing the second portion to form one or more ridges on an inner surface of the first portion when the second portion is overmoulded over the first portion. Also, the one or more ridges may be configured to be coupled to a mating structure arranged on the outer surface of the patient interface fitting. The second end may further comprise a ring-shaped seal being disposed in use between an end of the patient interface fitting and an annular flange of the first rigid portion on the inner surface of the second end. The second portion may correspond to one or more ridges disposed in one or more channels defined on an interior surface of the first portion. The one or more ridges may be configured to grip with and/or engage corresponding tabs arranged on the outer surface of the patient interface fitting. The one or more ridges and the corresponding tabs may form a seal when the second end and the patient interface fitting are coupled.

In an embodiment, the second end comprises a rigid material and one or more ridges on an inner surface.

In a further embodiment, the second end further comprises one or more gaps surrounding the one or more ridges, the one or more gaps enabling the one or more rigid ridges to flex so as to allow a mating structure arranged on the outer surface of the patient interface fitting to be coupled to the second end.

In an embodiment, the second end further comprises a ring-shaped seal being disposed in use between an end of the patient interface fitting and an annular flange on the inner surface of the second end.

In another embodiment, the second end comprises an opening, and/or an intermediate neck region, and/or a confined area. The opening may comprise an inner diameter varying from a first diameter proximal to the intermediate neck region to a second diameter distal from the intermediate neck region, the first diameter being less than the second diameter. The opening may be adapted to receive and guide the patient interface fitting during insertion into the second end. The intermediate neck region may be adapted to deform to allow passage of the patient interface fitting. The patient interface fitting may comprise a flanged end portion and the intermediate neck region is adapted to deform to allow passage of the flanged end portion. The confined area may be adapted to receive and retain the patient interface fitting when the Luer lock connector and the patient interface fitting are coupled. The patient interface fitting may comprise a flanged end portion and the confined area is adapted to receive and retain the flanged end portion when the Luer lock connector and the patient interface fitting are coupled. The intermediate neck portion may conform around an outer surface of the patient interface fitting to form a seal when the second end and the patient interface fitting are coupled. The seal may be formed only between the intermediate neck region and the outer surface of the patient interface fitting. The patient interface fitting may comprise a shaft portion and the intermediate neck portion conforms around an outer surface of the shaft portion to form a seal when the second end and the patient interface fitting are coupled. The second end may comprise an inner diameter which is larger or the same as an inner diameter of the patient interface fitting.

In a further embodiment, the second end comprises a flexible material and an inner diameter of the second end deforms and/or expands in cross section when the second end and the patient interface fitting are coupled, the deformation providing a sealing force forming a seal between the second end and the patient interface fitting.

In an embodiment, the second end is configured to lock around the outer surface of the patient interface fitting before, during or after engagement of the Luer lock connector with the patient interface.

In another embodiment, an inner diameter of an inner surface of the second end is less than an outer diameter of the patient interface fitting.

In a further embodiment, the second end comprises a first portion made of a rigid material and a second portion made of a flexible material. The first portion may comprise a first extremity attached to an outer surface of the second portion, the first extremity being adjacent to a distal end of the second end. The first portion may be shaped so as to form levers at a second extremity, the levers extending longitudinally along the gases flow passageway and being spaced apart and away from the outer surface of the second portion. The levers may be configured to be actuated by an operator so as to increase the inner diameter of the second end and allow the Luer lock connector to engage with the patient interface. The second portion of the second end may be configured to lock and seal around the outer surface of the patient interface fitting when the levers are released and the second end and the patient interface fitting are coupled. The first portion may be shaped so as to form levers, the levers extending radially and away from the gases flow passageway and being disposed on an outer surface of the first portion substantially opposite to the second portion. The levers may be configured to be actuated by an operator so as to increase the inner diameter of the second end and allow the Luer lock connector to engage with the patient interface. The first and second portions of the second end may be configured to lock and seal around the outer surface of the patient interface fitting when the levers are released and the second end and the patient interface fitting are coupled. An inner diameter of an inner surface of the second end may be greater than an outer diameter of the patient interface fitting. The second end may comprise a first portion made of a rigid material and a second portion made of a rigid material, the second portion further comprising a plurality of flexible inserts. The first portion may comprise a sleeve configured to be slid over the second portion by an operator so as to force the rigid portion and plurality of flexible inserts of the second portion to lock and seal around an outer surface of the patient interface fitting.

In an embodiment, the second end includes: a first portion made of a rigid material and comprising a first ring and a second ring, the second ring comprising a plurality of concave sections and being adapted to rotate relative to the first ring; and a second portion made of flexible material and comprising a plurality of flexible blades, each of the plurality of flexible blades being attached to the first ring and comprising at least one convex section adapted to receive one of the plurality of concave sections of the second ring. The flexible blades may be configured to lock and seal around an outer surface of the patient interface fitting when the first ring is rotated relative to the second ring by an operator.

In another embodiment, the second end comprises a flexible material and a fastening element. The fastening element may be configured to be actuated by an operator when the second end of the Luer lock connector is engaged with the patient interface so as to lock and seal the second end around an outer surface of the patient interface fitting. The fastening element may be one of: a velcro fastening assembly; a releasable ratchet tie-strap assembly; and a hook-and-loop fastening assembly.

In a further embodiment, the first end of the Luer connector described hereinabove is configured to be coupled to a dual-tubing conduit. The first end may comprise boss and barb connectors, the boss connector comprising a projection configured to be inserted within an outer tubing of the dual tubing conduit, and the barb connector comprising a projection configured to be inserted within an inner tubing of the dual-tubing conduit. The first end may comprise a connector having boss and barb portions, the barb portion being longitudinally offset from the boss portion and comprising a projection configured to be inserted within an inner conduit of the dual-tubing conduit, and an outer tubing of the dual-tubing conduit extends further toward the first end than the inner tubing. The second end of the Luer lock connector may be overmoulded onto the first end. The first end may comprise a cuff connector extending inside the dual-tubing conduit. The second end of the Luer connector may be overmoulded onto the cuff connector. Inner and outer tubings of the dual-tubing conduit may be pressed together and bonded to the cuff connector by the overmoulded second end.

In an embodiment, the first end is configured to be coupled to a single-tubing conduit. The first end may comprise a cuff connector extending inside the single-tubing conduit. The second end of the Luer connector may be overmoulded onto the cuff connector. The single-tubing conduit may be bonded to the cuff connector by the overmoulded second end. The single-tubing conduit may comprise annular, helical, or helical crested corrugations. The single-tubing conduit may comprise a helical bead and bubbles or a helical bead and a film.

In another embodiment, the Luer lock connector as described hereinabove, further comprises at least one sensor positioned in the gases flow passageway. The sensor may be configured to measure data relevant to one or more of the following: a temperature; humidity; a pressure; and a flow rate of the gases flow. The data may be transmitted to a remote apparatus via a wire associated with the tubing arrangement or via a flying lead. The data may be transmitted wirelessly to a remote apparatus. The data may be transmitted by radio-frequency identification or Wi-Fi.

In a further embodiment, the Luer lock connector as described hereinabove, further includes at least one non-return valve positioned in the gases flow passageway.

In an embodiment, the Luer lock connector as described hereinabove, further includes one or more portions made of a gas indicator material. The gas indicator material may comprise an indicator dye. The gas indicator material may be relevant to one or more of the following: a carbon dioxide concentration; humidity level; and temperature.

In a second aspect, the invention consists in a kit of parts for an unassembled insufflation system, the kit including: a tube defining a gases flow path from an outlet of a humidification chamber to a patient interface; and a Luer lock connector configured to removably couple an end of the tube to the patient interface, the Luer lock connector comprising: a first end configured to be coupled to the tube; and a second end configured to be coupled to a patient interface fitting of the patient interface, the second end being further configured to lock and seal around an outer surface of the patient interface fitting when the Luer lock connector is coupled to the patient interface.

In an embodiment, the kit of parts further includes a humidification chamber adapted to hold a volume of humidification liquid.

In another embodiment, the kit of parts further includes a supply tube, the supply tube defining a gases flow path from a gases source to an inlet of the humidification apparatus.

In a third aspect, the invention consists of an insufflation system including: a gases source; a patient interface; a delivery circuit defining a gases flow path between the gases source and the patient interface, the delivery circuit including at least one tube; and a Luer lock connector configured to removably couple an end of the tube to the patient interface, the Luer lock connector comprising: a first end configured to be coupled to the tube; and a second end configured to be coupled to a patient interface fitting of the patient interface, the second end being further configured to lock and seal around an outer surface of the patient interface fitting when the Luer lock connector is coupled to the patient interface.

In an embodiment, the insufflation system further includes a humidification apparatus, the humidification apparatus being disposed in use in the gas delivery circuit between the gases source and the patient interface. The at least one tube may comprise a first tube configured to couple the humidification apparatus to the patient interface. The at least one tube may comprise a second tube configured to couple the humidification apparatus to the gases source.

In another embodiment, the patient interface includes a trocar or cannula for laparoscopic surgery.

In a further embodiment, the patient interface includes a diffuser for open surgery.

In an embodiment, the gases source includes a carbon dioxide supply.

In a fourth aspect, the invention consists in an insufflation system including: a gases source; a patient interface comprising an outer surface having a flanged end portion and a shaft portion; a delivery circuit defining a gases flow path between the gases source and the patient interface, the delivery circuit including at least one tube; and a Luer lock connector configured to be: removably coupled to the at least one tube at a first end; and, coupled to the flanged end and shaft portions of the patient interface at a second end such that the Luer lock connector locks and seals around the outer surface of the patient interface when coupled to the patient interface. The insufflation system of the fourth aspect may be used with any of the aspects and embodiments described hereinabove.

In a fifth aspect, the invention consists in a connector assembly including: a patient interface connector configured to be coupled to or with a patient interface, the patient interface connector comprising an outer surface having a flanged end portion and a shaft portion; and a Luer lock connector comprising first and second portions, the first portion being configured to be coupled to or with a tubing arrangement, and the second portion being configured to be coupled to or with the patient interface connector; wherein, the second portion of the Luer lock connector comprises a confined area and an intermediate neck region, and wherein the flanged end portion of the patient interface connector is configured to be received and retained in the confined area and the intermediate neck region is configured to seal around an outer surface of the shaft portion when the Luer lock connector is coupled to the patient interface connector. The connector assembly of the fifth aspect may be used with any of the aspects and embodiments described hereinabove.

In a sixth aspect, the invention consists in a Luer lock connector for use in an insufflation system, the Luer lock connector including: a first portion configured to be coupled to or with a tubing arrangement; and a second portion to provide for a seal with a patient interface fitting; wherein, the first portion and the second portion define an interior region forming a gases flow passageway allowing insufflation gases to flow through the Luer lock connector from a first end to a second end; and wherein, the second portion is configured to seal around an outer surface of the patient interface fitting when the Luer lock connector is coupled to the patient interface.

In an embodiment, the second portion is overmoulded over the first portion.

In another embodiment, the first portion comprises a first material and the second portion comprises a second material, the second material being softer or more flexible than the first material.

In a further embodiment, the second portion comprises one or more ridges located on an internal surface of the second portion. The one or more ridges may define a neck region to create the seal around the outer surface of the patient interface, optionally the neck region is substantially circular and optionally of a smaller diameter than the patient interface fitting.

In an embodiment, the Luer lock connector of the sixth aspect may be used in conjunction with any of the aspects and embodiments described hereinabove.

In a seventh aspect, the present invention consists in a Luer lock connector for use in an insufflation system, the Luer lock connector comprising: a body comprising a first end, a second end and an interior region; the interior region defining a gases flow passageway allowing insufflation gases to flow through the body from the first end to the second end; and the body being configured to be coupled to a tubing arrangement at the first end and to a patient interface at the second end; wherein, the second end is configured to be coupled to a patient interface fitting of the patient interface, the second end being further configured to seal around an outer surface of the patient interface fitting when the Luer lock connector is coupled to the patient interface; and wherein, the second end forms a seal with the outer surface of the patient interface fitting when the second end and the patient interface fitting are coupled.

In an embodiment, the seal between the second end and the outer surface of the patient interface fitting is the only seal between the patient interface and the second end.

In another embodiment, the second end comprises an opening, and/or a neck region, and/or a confined area, optionally the neck portion is intermediate of the opening, and the confined area.

In a further embodiment, the opening comprises an inner diameter varying from a first diameter proximal to the neck region to a second diameter distal from the neck region, the first diameter being less than the second diameter.

In an embodiment, the opening is adapted to receive and guide the patient interface fitting during insertion into the second end.

In another embodiment, the neck region is adapted to deform to allow passage of the patient interface fitting.

In a further embodiment, the patient interface fitting comprises a flanged end portion and the neck region is adapted to deform to allow passage of the flanged end portion.

In an embodiment, the confined area is adapted to receive and retain the patient interface fitting when the Luer lock connector and the patient interface fitting are coupled.

In another embodiment, the patient interface fitting comprises a flanged end portion and the confined area is adapted to receive and retain the flanged end portion when the Luer lock connector and the patient interface fitting are coupled

In a further embodiment, the neck portion conforms around an outer surface of the patient interface fitting to form a seal when the second end and the patient interface fitting are coupled.

In an embodiment, the seal is formed only between the neck region and the outer surface of the patient interface fitting.

In another embodiment, the patient interface fitting comprises a shaft portion and the neck portion conforms around an outer surface of the shaft portion to form the seal when the second end and the patient interface fitting are coupled, optionally the seal is provided along a length of a shaft of the patient interface connector.

In a further embodiment, the second end comprises an inner diameter which is larger or the same as an inner diameter of the patient interface fitting.

In another embodiment, the patient interface fitting is configured to be inserted into the second end of the Luer lock connector.

In a further embodiment, the second end comprises a flexible material and one or more ridges on an inner surface.

In an embodiment, the second end is configured to be more flexible or softer than the first end.

In another embodiment, the one or more ridges are configured to be coupled to a mating structure arranged on the outer surface of the patient interface fitting.

In a further embodiment, the one or more ridges and the mating structure form the seal when the second end and the patient interface fitting are coupled.

In an embodiment, the second end further comprises a distal end having an inner diameter that is less than an outer diameter of the patient interface fitting, and wherein the distal end presses onto the outer surface of the patient interface fitting to form a seal when the second end and the patient interface fitting are coupled.

In another embodiment, the second end comprises a first portion made of a rigid material and a second portion made of a flexible material.

In a further embodiment, the second portion is overmoulded over the first portion and the first portion comprises one or more gaps allowing the second portion to form one or more ridges on an inner surface of the first portion when the second portion is overmoulded over the first portion.

In an embodiment, the second end comprises a flexible material and an inner diameter of the second end deforms and/or expands in cross section when the second end and the patient interface fitting are coupled, the deformation providing a sealing force forming the seal between the second end and the patient interface fitting.

In another embodiment, an inner diameter of an inner surface of the second end is less than an outer diameter of the patient interface fitting.

In a further embodiment, the first end is configured to be coupled to a dual-tubing conduit. The first end may comprise boss and barb connectors, the boss connector comprising a projection configured to be inserted within an outer tubing of the dual tubing conduit, and the barb connector comprising a projection configured to be inserted within an inner tubing of the dual-tubing conduit. Alternatively, the first end may comprise a connector having boss and barb portions, the barb portion being longitudinally offset from the boss portion and comprising a projection configured to be inserted within an inner conduit of the dual-tubing conduit, and an outer tubing of the dual-tubing conduit extends further toward the first end than the inner tubing.

In an eight aspect, the invention consists in a Luer lock connector for use in an insufflation system, the Luer lock connector comprising: a body comprising a first end, a second end and an interior region; the interior region defining a gases flow passageway allowing insufflation gases to flow through the body from the first end to the second end; and the body being configured to be coupled to a tubing arrangement at the first end and to a patient interface at the second end; wherein, the second end is configured to be coupled to a patient interface fitting of the patient interface, the second end being further configured to seal around an outer surface of the patient interface fitting when the Luer lock connector is coupled to the patient interface; and wherein, the second end forms a seal with the outer surface of the patient interface fitting when the second end and the patient interface fitting are coupled; and wherein, the second end comprises a neck region adapted to deform to allow passage of the patient interface fitting.

In one embodiment, the deformation of the neck region is configured to form the seal with the outer surface of the patient interface fitting.

In another embodiment, the second end further comprises a ring-shaped seal being disposed in use between an end of the patient interface fitting and an annular flange on an inner surface of the second end, optionally the ring-shaped seal is provided at least in part by a surface of the second end.

In a further embodiment, the second end further comprises an opening, and/or a confined area, optionally the neck portion is intermediate of the opening, and the confined area.

In an embodiment, the opening comprises an inner diameter varying from a first diameter proximal to the neck region to a second diameter distal from the neck region, the first diameter being less than the second diameter.

In another embodiment, the opening is adapted to receive and guide the patient interface fitting during insertion into the second end.

In a further embodiment, the neck region is adapted to deform to allow passage of the patient interface fitting.

In one embodiment, the patient interface fitting comprises a flanged end portion and the neck region is adapted to deform to allow passage of the flanged end portion.

In another embodiment, the confined area is adapted to receive and retain the patient interface fitting when the Luer lock connector and the patient interface fitting are coupled.

In a further embodiment, the patient interface fitting comprises a flanged end portion and the confined area is adapted to receive and retain the flanged end portion when the Luer lock connector and the patient interface fitting are coupled.

In an embodiment, the neck portion conforms around an outer surface of the patient interface fitting to form the seal when the second end and the patient interface fitting are coupled.

In another embodiment, the seal is formed only between the neck region and the outer surface of the patient interface fitting.

In a further embodiment, the patient interface fitting comprises a shaft portion and the neck portion conforms around an outer surface of the shaft portion to form the seal when the second end and the patient interface fitting are coupled, optionally the seal is provided along a length of a shaft of the patient interface connector.

In an embodiment, the second end comprises an inner diameter which is larger or the same as an inner diameter of the patient interface fitting.

In another embodiment, the patient interface fitting is configured to be inserted into the second end of the Luer lock connector.

In a further embodiment, the second end comprises a flexible material and one or more ridges on an inner surface.

In an embodiment, the second end is configured to be more flexible or softer than the first end.

In another embodiment, the one or more ridges are configured to be coupled to a mating structure arranged on the outer surface of the patient interface fitting.

In a further embodiment, the one or more ridges and the mating structure form the seal when the second end and the patient interface fitting are coupled.

In an embodiment, the second end further comprises a distal end having an inner diameter that is less than an outer diameter of the patient interface fitting, and wherein the distal end presses onto the outer surface of the patient interface fitting to form a seal when the second end and the patient interface fitting are coupled.

In another embodiment, the second end comprises a first portion made of a rigid material and a second portion made of a flexible material.

In a further embodiment, the second portion is overmoulded over the first portion and the first portion comprises one or more gaps allowing the second portion to form one or more ridges on an inner surface of the first portion when the second portion is overmoulded over the first portion.

In an embodiment, the second end comprises a flexible material and an inner diameter of the second end deforms and/or expands in cross section when the second end and the patient interface fitting are coupled, the deformation providing a sealing force forming the seal between the second end and the patient interface fitting.

In another embodiment, an inner diameter of an inner surface of the second end is less than an outer diameter of the patient interface fitting.

In a further embodiment, the first end is configured to be coupled to a dual-tubing conduit. The first end may comprise boss and barb connectors, the boss connector comprising a projection configured to be inserted within an outer tubing of the dual tubing conduit, and the barb connector comprising a projection configured to be inserted within an inner tubing of the dual-tubing conduit. Alternatively, the first end may comprise a connector having boss and barb portions, the barb portion being longitudinally offset from the boss portion and comprising a projection configured to be inserted within an inner conduit of the dual-tubing conduit, and an outer tubing of the dual-tubing conduit extends further toward the first end than the inner tubing.

In a ninth aspect, the invention consists in a Luer lock connector for use in an insufflation system, the Luer lock connector comprising: a body comprising a first end, a second end and an interior region; the interior region defining a gases flow passageway allowing insufflation gases to flow through the body from the first end to the second end; and the body being configured to be coupled to a tubing arrangement at the first end and to a patient interface at the second end; wherein, the second end is configured to be coupled to a patient interface fitting of the patient interface, the second end being further configured to create a single seal around an outer surface of the patient interface fitting when the Luer lock connector is coupled to the patient interface; and wherein, the second end forms the single seal with the outer surface of the patient interface fitting when the second end and the patient interface fitting are coupled.

In an embodiment, the Luer lock connector of the ninth aspect may be used in conjunction with any of the aspects and embodiments described hereinabove.

In a tenth aspect, the invention consists in a connector assembly comprising: a patient interface connector configured to be coupled to or with a patient interface, the patient interface connector comprising an outer surface having a flanged end portion and a shaft portion; a Luer lock connector comprising first and second portions, the first portion being configured to be coupled to or with a tubing arrangement, and the second portion being configured to be coupled to or with the patient interface connector; wherein, the second portion of said Luer lock connector comprises a confined area and a neck region, and wherein the flanged end portion of the patient interface connector is configured to be received and retained in the confined area and the neck region is configured to seal around an outer surface of the shaft portion when the Luer lock connector is coupled to said patient interface connector.

In an embodiment, the Luer lock connector of the tenth aspect may be used in conjunction with any of the aspects and embodiments described hereinabove.

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting.

### BRIEF DESCRIPTION OF THE DRAWINGS

One preferred form of the present invention will now be described with reference to the accompanying drawings in which:
Figure 1 is a schematic view of an insufflation system embodying a Luer connector, constructed and operative in accordance with an embodiment of the present invention;
Figure 2 is a side cross sectional view of a first end of a Luer connector, constructed and operative in accordance with an embodiment of the present invention;
Figure 3 is a side cross sectional view of a first end of a Luer connector, constructed and operative in accordance with another embodiment of the present invention;
Figure 4 is a side cross sectional view of a first end of a Luer connector, constructed and operative in accordance with a further embodiment of the present invention;
Figures 5A-5I are side cross sectional views of a first end of a Luer connector, constructed and operative in accordance with other embodiments of the present invention;
Figure 6 is a side cross sectional view of a first end of a Luer connector, constructed and operative in accordance with a further embodiment of the present invention;
Figures 7A-7B are side cross sectional views of a first end of a Luer connector, constructed and operative in accordance with other embodiments of the present invention;
Figures 8A-8C are side cross sectional views of a first end of a Luer connector, constructed and operative in accordance with further embodiments of the present invention;
Figure 9A is a side cross sectional view of a first end of a Luer connector, constructed and operative in accordance with another embodiment of the present invention;
Figure 9B is an isometric view of the first end of the Luer connector of Fig. 9A;
Figure 9C is a left view of the first end of the Luer connector of Fig. 9A;
Figure 10A is side view of a first end of a Luer connector, constructed and operative in accordance with a further embodiment of the present invention;
Figure 10B is a bottom view of the first end of the Luer connector of Fig. 10A;
Figure 10C is a left view of the first end of the Luer connector of Fig. 10A;
Figure 11 is a side view of a first end of a Luer connector, constructed and operative in accordance with another embodiment of the present invention;
Figures 12A-12D are different view of a first end of a Luer connector, constructed and operative in accordance with a further embodiment of the present invention;
Figure 13 is a side view of a first end of a Luer connector, constructed and operative in accordance with another embodiment of the present invention;
Figure 14 is a side cross sectional view of a second end of a Luer connector, constructed and operative in accordance with an embodiment of the present invention;
Figures 15A and 15B are side cross sectional views of a second end of a Luer connector, constructed and operative in accordance with other embodiments of the present invention;
Figure 16 is a side cross sectional view of a second end of a Luer connector, constructed and operative in accordance with a further embodiment of the present invention;
Figure 17 is a side cross sectional view of a second end of a Luer connector, constructed and operative in accordance with another embodiment of the present invention;
Figure 18 is a side cross sectional view of a Luer connector including a sensor, constructed and operative in accordance with an embodiment of the present invention;
Figure 19 is a side cross sectional view of a Luer connector including a non-return valve, constructed and operative in accordance with another embodiment of the present invention;
Figure 20 is a side cross sectional view of Luer connector including a gas indicator material, constructed and operative in accordance with a further embodiment of the present invention.
Figures 21A-21F are side cross sectional views of a second end of a Luer connector connected to different tube arrangements, constructed and operative in accordance with embodiments of the present invention;
Figure 22A is a side cross sectional view of a Luer connector, constructed and operative in accordance with an embodiment of the present invention;
Figure 22B is a side cross sectional view of the Luer connector of Fig. 22A coupled to a patient interface fitting;
Figure 22C is a side cross sectional view of the Luer connector of Figs. 22A and 22B coupled to a conduit;
Figure 22D is a side view of the Luer connector of Figs. 22A to 22C; and
Figure 22E is an isometric view of the Luer connector of Figs. 22A to 22D.

### DETAILED DESCRIPTION

In the following description, numerous specific details are set forth in order to provide a thorough understanding of the various principles of the present invention. However, those skilled in the art will appreciate that not all these details are necessarily always required for practicing the present invention.

Although the principles of the present invention are largely described herein in relation to laparoscopy or open surgery procedures, this is an example selected for convenience of presentation, and is not limiting. The Luer connectors described herein may be used for any suitable medical procedure and in any suitable medical system comprising a gas delivery circuit.

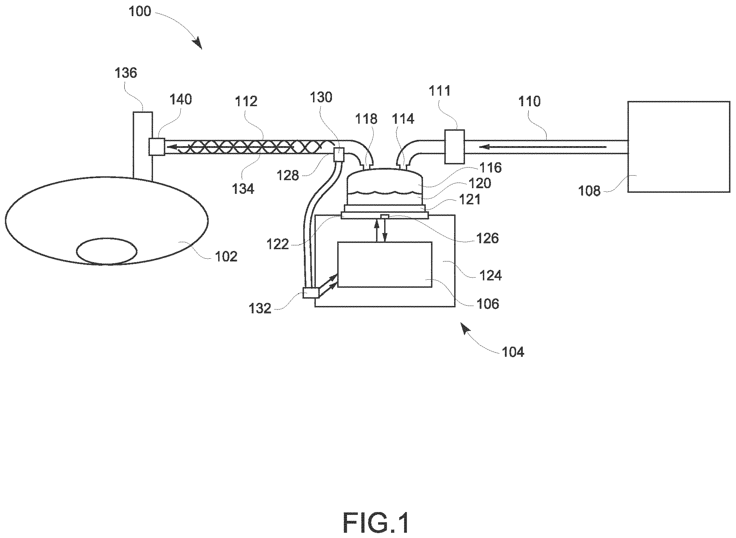

Reference is now made to Fig. 1, which is a schematic view of an insufflation system embodying a Luer connector constructed and operative in accordance with an embodiment of the present invention.

Fig. 1 illustrates an insufflation system 100 for delivering temperature- and humidity-controlled gas to a patient 102, the insufflation system 100 having a humidification apparatus or humidifier 104 incorporating a humidifier control system 106. The humidifier 104 is connected to a gas source 108 through an inlet conduit 110. The humidifier 104 delivers humidified gas to the patient 102 through a patient conduit 112. The conduits 110, 112 can be made of flexible plastic tubing.

The humidifier 104 receives gas from the gas source 108 through the inlet conduit 110. The gas can be filtered through a filter 111 and delivered to the humidifier 104 through a humidifier inlet 114. The gas is humidified as it passes through a humidifying chamber 116, which is effectively a water bath, and the gas flows out through a humidifier outlet 118 and into the patient conduit 112. The gas then moves through the patient conduit 112 to the patient 102 via the Luer connector 140 and the patient interface 136. The patient interface 136 may be, for example, but not limited to, a trocar or cannula for laparoscopic surgery or a diffuser for open surgery.

The humidifier 104 comprises a body 124 removably engageable with the humidification chamber 116. The humidification chamber 116 has a metal base 121 and is adapted to hold a volume of water 120, which can be heated by a heater plate 122. The heater plate 122 can be in thermal contact with the metal base 121 of the humidification chamber 116. Providing power to the heater plate 122 can cause heat to flow from the heater plate 122 to the water 120 through the metal base 121. As the water 120 within the humidification chamber 116 is heated it can evaporate and the evaporated water can mix with gases flowing through the humidification chamber 116 from the filter 111 and gas source 108. Accordingly, the humidified gases leave the humidification chamber 116 via outlet 118 and are passed to the patient 102 via the patient conduit 112, the Luer connector 140, the patient interface 136 and into the surgical site to, for example, insufflate the surgical site and/or expand body cavity.

The humidifier 104 includes the humidifier control system 106 configured to control a temperature and/or humidity of the gas being delivered to the patient 102. The humidifier control system 106 can be configured to regulate an amount of humidity supplied to the gases by controlling an electrical power supplied to the heater base 122. The humidifier control system 106 can control operation of the humidification system 104 in accordance with instructions set in software and in response to system inputs. System inputs can include a heater plate sensor 126, an outlet chamber temperature sensor 128, and a chamber outlet flow sensor 130. For example, the humidifier control system 106 can receive temperature information from the heater plate sensor 126 which it can use as an input to a control module used to control the power or temperature set point of the heater plate 122. The humidifier control system 106 can be provided with inputs of temperature and/or flow rates of the gases. For example, the chamber outlet temperature sensor 128 can be provided to indicate to the humidifier control system 106 the temperature of the humidified gas as it leaves the outlet 118 of the humidification chamber 116. The temperature of the gases exiting the chamber can be measured using any suitable temperature sensor 128, such as a wire-based temperature sensor. The chamber outlet flow sensor 130 can be provided to indicate to the humidifier control system 106 the flow rate of the humidified gas. The flow rate of the gases through the chamber 116 can be measured using any suitable flow sensor 130, such as a hot wire anemometer. In some embodiments, the temperature sensor 128 and flow sensor 130 are in the same sensor housing. The temperature sensor 128 and flow sensor 130 can be connected to the humidifier 104 via connector 132. Additional sensors may be incorporated into the insufflation system 100, for example, for sensing parameters at the patient end of the patient conduit 112.

The humidifier control system 106 can be in communication with the heater plate 122 such that the humidifier control system 106 can control a power delivered to the heater plate 122 and/or control a temperature set point of the heater plate 122. As described further herein, the humidifier control system 106 can determine an amount of power to deliver to the heater plate 122, or a heater plate set point, based at least in part on a flow condition, an operation mode, a flow reading, an outlet temperature reading, a heater plate sensor reading, or any combination of these or other factors.

The insufflation system 100 can include a conduit heating wire 134 configured to provide heat to the gases traveling along the patient conduit 112. Gases leaving the outlet 118 of the humidification chamber 116 can have a high relative humidity (e.g., about 100%). As the gases travel along the patient conduit 112 there is a chance that water vapor may condense on the conduit wall, reducing the water content of the gases. To reduce condensation of the gases within the conduit, the conduit heating wire 134 can be provided within, throughout, and/or around the patient conduit 112. Power can be supplied to the conduit heating wire 134 from the humidifier 104 and can be controlled through the humidifier control system 106. In some embodiments, the heating wire 134 is configured to maintain the temperature of the gas flowing through the patient conduit 112. In some embodiments, the conduit heating wire 134 can be configured to provide additional heating of the gas to elevate the gases temperature to maintain the humidity generated by the heated water bath in the humidifier 104.

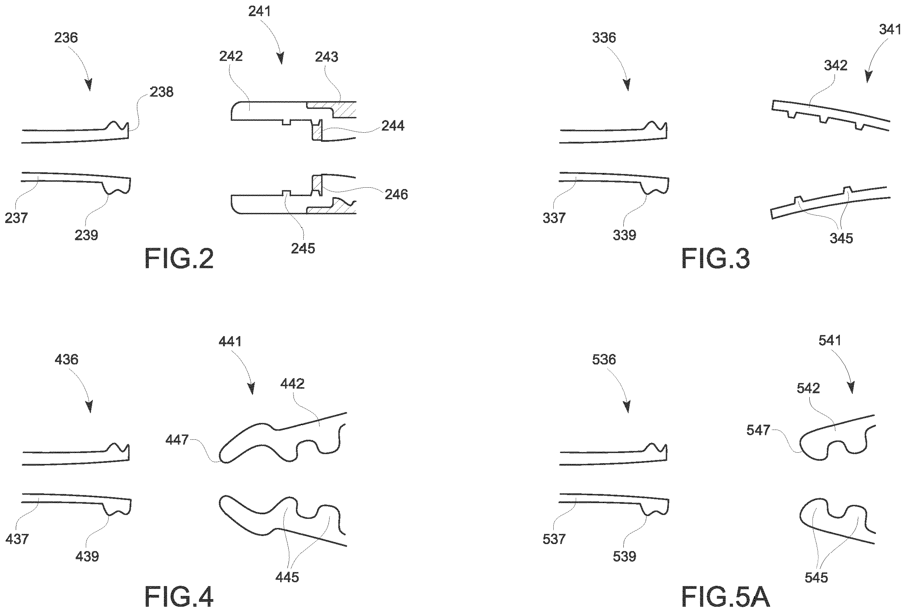

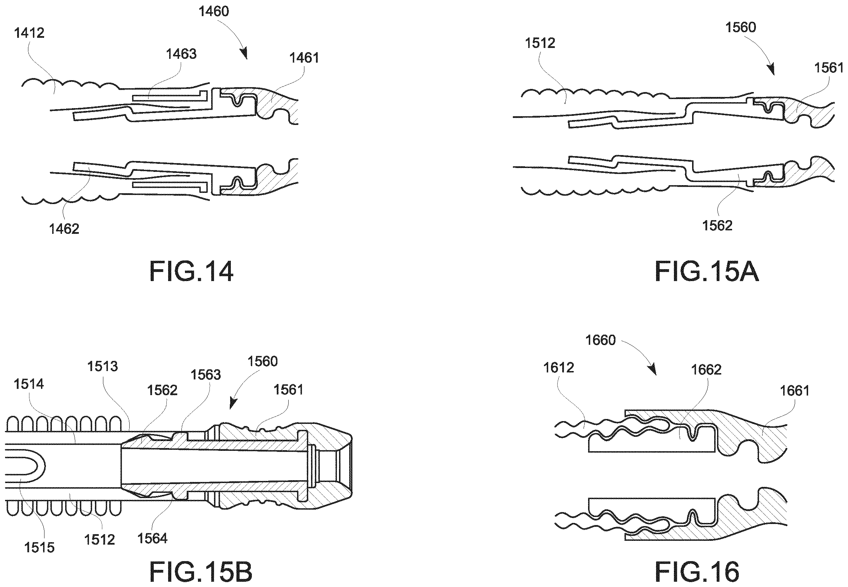

The Luer connector 140 may comprise a body having an interior region defining a gases flow passageway allowing insufflation gases to flow through. The body comprises a first end, hereinafter referred as the tubing end, which permanently attaches to the tubing of the patient conduit 112, and a second end, hereinafter referred as the Luer end, which removably connects to a fitting of the patient interface 136. It will be appreciated that the Luer connector 140 of Fig. 1 is a high flow Luer connector as it provides particular sealing and retention features with less resistance to gases flow than traditional Luer connectors of the art. Figs. 2-13 and 22A-22E illustrate several alternate embodiments for the Luer end of the Luer connector 140 of the present invention. Similarly, Figs. 14-21F and 22A-22E illustrate several alternate embodiments for the tubing end of the Luer connector 140 of the present invention. It will be appreciated by those skilled in the art that the high flow Luer connector 140 of the present invention may comprise any suitable combination of Luer and tubing ends as depicted in Figs. 2 to 21F so as to provide sealing and retention features with less resistance to gases flow than the traditional Luer connectors of the art.

Reference is now made to Fig. 2, which is a side cross sectional view of a first end of a Luer connector, constructed and operative in accordance with an embodiment of the present invention.

Fig. 2 illustrates a Luer end 241 of a high flow Luer connector adapted to receive a corresponding end of the patient interface 236. The Luer end 241 may comprise a rigid first portion 242 having threads 245 (e.g. helical threads) on an inner surface and a flexible second portion 243 overmoulded around an outer surface of the first portion 242. The flexible second portion 243 may be made of any suitable flexible material such as, for example, but not limited to, a flexible thermoset elastomer or thermoplastic. By contrast, the rigid first portion 242 may be made of any suitable material such as, for example but not limited to, a rigid thermoset or thermoplastic material. The Luer end 241 may further comprise a ring-shaped gasket 244 positioned on a shoulder 246 formed on an inner surface of the rigid first portion 242. Additionally, and/or alternatively, the rigid first portion 242 may comprise small gaps (not shown) around its outer surface enabling the material of the flexible second portion 243 to form the ring-shaped gasket 244 during the overmoulding process. The patient interface fitting 237 comprises a distal end 238 and external tabs 239 (e.g. threads and/or helical threads) positioned on an outer surface. The threads 245 and tabs 239 are configured to be coupled so as to enable the rigid first portion 242 of the Luer connector to lock around the outer surface of the patient interface fitting 237. When connected, the distal end 238 of the patient interface fitting 237 abuts onto the ring-shaped gasket 244 of the Luer connector thereby providing a tight seal between the Luer connector and the patient interface 236.

Reference is now made to Fig. 3, which is a side cross sectional view of a first end of a Luer connector, constructed and operative in accordance with another embodiment of the present invention.

Fig. 3 illustrates a Luer end 341 of a high flow Luer connector adapted to receive a corresponding end of the patient interface 336. The Luer end 341 may comprise a semi-rigid portion 342 having threads 345 on an inner tapered surface that becomes smaller in diameter closer to the tubing end (not shown) of the Luer connector. The semi-rigid portion 342 may be made of any suitable semi-rigid material such as, for example, but not limited to, a flexible thermoset elastomer or thermoplastic. It will be appreciated by those skilled in the art that the flexible thermoset elastomer or thermoplastic suitable for use for the semi-rigid portion 342 may have different properties as the ones suitable for use for the flexible second portion 243 of Fig. 2. The patient interface fitting 337 may comprise external tabs 339 positioned on an outer surface. The threads tabs 345 and tabs 339 are configured to be coupled so as to enable the semi-rigid portion 342 of the Luer connector to lock around the outer surface of the patient interface fitting 337. When the Luer end 341 of the Luer connector is spun onto the patient interface fitting 337, the semi-rigid portion 342 conforms around the outer surface of the patient interface fitting 337 thereby providing a tight lock and seal between the Luer connector and the patient interface 336.

Reference is now made to Fig. 4, which is a side cross sectional view of a first end of a Luer connector, constructed and operative in accordance with a further embodiment of the present invention.

Fig.4 illustrates a Luer end 441 of a high flow connector adapted to receive a corresponding end of the patient interface 436. The Luer end 441 may comprise a flexible portion 442 having ridges 445 on an inner surface. The flexible portion 442 may be made of any suitable flexible material such as, for example, but not limited to, a flexible thermoset elastomer or thermoplastic. The patient interface fitting 437 of the patient interface 436 comprises tabs 439 positioned on an outer surface. The tabs 439 and ridges 445 have complementary shapes so as to enable the flexible portion 442 of the Luer connector to lock around the outer surface of the patient interface fitting 437. In addition, the inner diameter of the Luer end 441 is smaller than the outer diameter of the patient interface fitting 437 thereby providing a push-on attachment to the patient interface fitting 437. In other words, the patient interface fitting 437 is pushed against a distal end 447 of the Luer end (or vice versa) so as to initiate the connection between the patient interface 436 and the Luer connector. When the patient interface fitting 437 is pushed further, the flexible portion 442 deforms so as to allow the tabs 439 to reach and interlock with the ridges 445. When connected, the distal end 447 of the Luer end presses the outer surface of the patient interface fitting 437 thereby providing a tight seal between the Luer connector and the patient interface 436. It will be appreciated that the locking mechanism formed by the tabs 439 and the ridges 445 also contributes to create a seal.

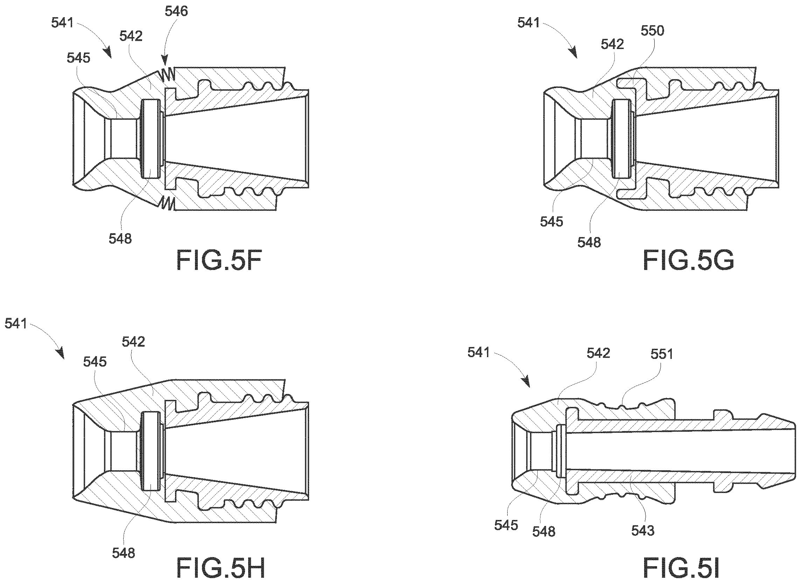

Reference is now made to Figs. 5A-5H, which are side cross sectional views of a first end of a Luer connector, constructed and operative in accordance with embodiments of the present invention.

Fig. 5A shows an embodiment of the present invention which is largely similar to the one illustrated in Fig. 4. The Luer end 541 may comprise a flexible portion 542 having ridges 545 on an inner surface. The patient interface fitting 537 of the patient interface 536 comprises tabs 539 positioned on an outer surface. The tabs 539 and threads 545 have complementary shapes so as to enable the flexible portion 542 of the Luer connector to lock around the outer surface of the patient interface fitting 537. However, the ridges 545 are provided adjacent to the distal end 547 of the Luer end. In such embodiment, the sealing mechanism is provided by the locking or retention mechanism formed by the tabs 539 and the ridges 545.

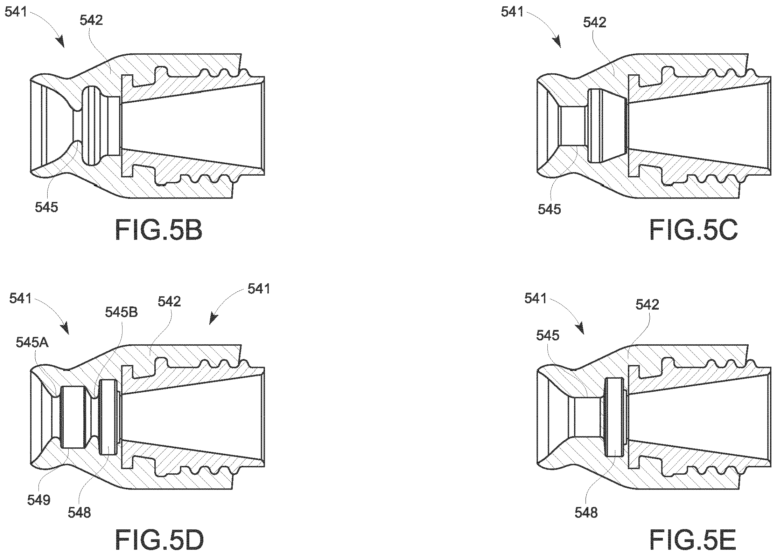

Figs. 5B-5I illustrate different embodiments of the present invention similar to the one shown in Fig. 5A with additional and/or alternate features. For example, the flexible portion 542 of:
- Fig. 5B comprises a wide and long tapered inlet facilitating the insertion of the patient interface fitting into the Luer connector. The flexible portion 542 may further comprise a ridge 545 which creates a narrow deforming inlet that seals around the outer surface of the patient interface fitting and also reduces the force required to pull on/off the patient interface fitting into the Luer connector;
- Fig. 5C comprises a long ridge 545 having an extended flat section operative to seal around the outer surface of the patient interface fitting and to improve the resistance to pulling/tugging. The flexible portion 542 may further comprise a tapered transition to the body and/or tubing end of the Luer connector which is provided to: mitigate the effects of the patient interface fitting moving around inside when forces are applied from the sides; and reduce the change occlusion;
- Fig. 5D comprises two ridges 545a and 545b, a transition area 549 between the two ridges 545a and 545b, and a confined area 548 for the patient interface fitting adjacent the second ridge 545b. The two ridges 545a and 545b provide two points of contact configured to seal around an outer surface of the patient interface fitting and also improve the resistance to pulling/tugging without obstructing the gases flow path. The confined area 548 is configured to receive tabs located on a distal end of the patient interface fitting and force these tabs to abut against the Luer connector, thereby reducing the risk of obstructing the gases flow path;
- Fig. 5E comprises a long and tight ridge 545 to seal around an outer surface of the patient interface fitting and a confined area 548 for the patient interface fitting adjacent the ridge 545. The confined area 548 is configured to receive tabs located on a distal end of the patient interface fitting and force these tabs to abut against the Luer connector, thereby reducing the risk of obstructing the gases flow path when the Luer connector is pulled/tugged.;
- Fig. 5F comprises slits 546 on an outer surface to ease the insertion of the patient interface fitting. In operation, the slits 546 facilitate the bending of the Luer end 541 to occur on the outside of the Luer connector, thereby avoiding the risk of occlusion of the gases flow path when forces are applied from the sides.
- Figs. 5G and 5H are similar to the one depicted on Figs. 5E and 5F. However, Fig. 5G shows an additional rim 550 configured to provide more rigidity to the Luer connector when forces are applied from the sides. In addition, the gases flow path is wider than adjacent to the confined area 548 thereby reducing the risk of occlusion when forces are applied from the sides of the Luer connector. Fig. 5H shows a flexible portion 542 with a tapered outer surface which does not comprise an external lip as shown in the Figs. 5B to 5G. This arrangement improves the rigidity of the Luer connector and reduces the risk of occlusion of the gases flow path when forces are applied from the sides; and [00160] Fig. 51 is similar to the ones depicted on Figs. 5E to 5H. The flexible portion 542 of the Luer end 541 comprises a ridge 545 (e.g. long and tight neck portion) to seal around an outer surface of the patient interface fitting, thereby creating a single seal between the flexible portion 542 and the outer surface of the patient interface fitting. In other words, a seal is formed only between the Luer end 541 of the flexible portion 542 and the outer surface of the patient interface fitting. The flexible portion 542 may further comprise a confined area 548 for the patient interface fitting adjacent the ridge 545. The confined area 548 is configured to receive and retain a flanged portion (e.g. tabs) located on a distal end of the patient interface fitting. The confined area may also force the flanged portion to abut against the Luer connector, for example a surface, and/or a flange of the rigid portion 543. By receiving and retaining the flanged portion of the patient interface connector the confined area 548 reduce the risk of obstructing the gases flow path when the Luer connector is pulled/tugged. By contrast to Figs. 5D-5H, the flexible portion 542 of the Luer end 541 is overmoulded onto the rigid portion 543 such that no material is provided between the confined area 548 and the rigid portion. Alternatively, the flexible portion 542 may be overmoulded onto the rigid portion 543 such that material is provided between the confined area 548 and the rigid portion 543. The material provided between the confined area 548 and the rigid portion may define a ring-shaped gasket 244 as described above. This latter arrangement creates a further seal (in addition to the one provided by the neck portion or ridge 545) between the flanged portion of the patient interface fitting and the flexible portion 542 of the Luer connector. In addition, Fig. 51 shows a flexible portion 542 with a tapered outer surface which does not comprise an external lip as shown in the Figs. 5B to 5G. This arrangement improves the rigidity of the Luer connector and reduces the risk of occlusion of the gases flow path, and/or reduces the risk of disconnection from the patient interface when forces are applied from the sides. Also, gripping means 551 (e.g. finger grips) may be provided on the outer surface of the flexible portion 542 so as to facilitate manipulation and use of the Luer connector.

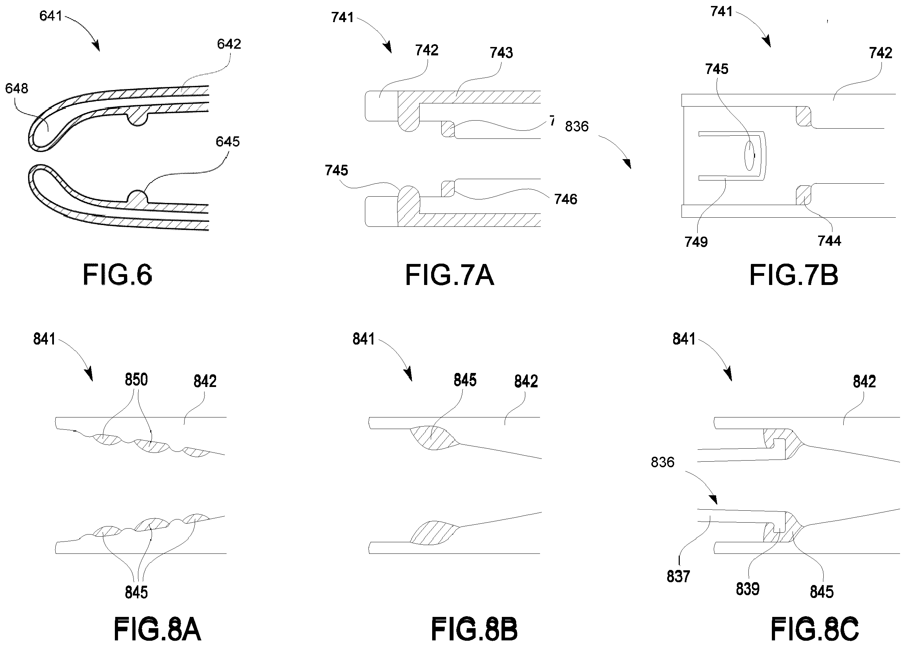

Reference is now made to Fig. 6, which is a side cross sectional view of a first end of a Luer connector, constructed and operative in accordance with a further embodiment of the present invention.

Fig. 6 illustrates a Luer end 641 of a high flow connector adapted to receive a corresponding end of a patient interface (not shown). The Luer end 641 may comprise a flexible and hollow portion 642 having ridges 645 on an inner surface. The inner diameter of the Luer end 641 is smaller than an outer diameter of a patient interface fitting (not shown) thereby providing a push-on attachment similar to the embodiments illustrated in Figs. 4 and 5A-5H. However, the flexible and hollow portion 642 further defines a cavity 648 that is adapted to receive a pressurized gas flow. When the patient interface and the Luer connector are connected, the cavity 648 may be filled with some gases so that the distal end 647 of the Luer end 641 expands/inflates and the seal strength around the outer surface of the patient interface fitting (not shown) is increased.

Reference is now made to Figs. 7A and 7B, which are cross sectional views of a first end of a Luer connector, constructed and operative in accordance with embodiments of the present invention.

Fig. 7A illustrates a Luer end 741 of a high flow Luer connector adapted to receive a corresponding end of the patient interface (not shown). The Luer end 741 may comprise a rigid first portion 742 and a flexible second portion 743 overmoulded around an outer surface of the rigid first portion 742. The rigid first portion 742 may comprise gaps (not shown) around its outer surface so that the flexible second portion 743 protrudes and forms ridges 745 on an inner surface of the Luer end 741. The flexible second portion 743 may be made of any suitable flexible material such as, for example, but not limited to, a flexible thermoset elastomer or thermoplastic. By contrast, the rigid first portion 742 may be made of any suitable material such as, for example but not limited to, a rigid thermoset or thermoplastic material. The Luer end 741 may further comprise a flexible ring-shaped gasket 744 positioned on a shoulder 746 formed on an inner surface of the rigid first portion 742. The patient interface fitting (not shown) comprises tabs positioned on an outer surface and adapted to engage the ridges 745 of the flexible second portion 743. A push-on attachment is therefore provided so that the Luer end 741 locks around the outer surface of the patient interface fitting. When connected, a distal end of the patient interface fitting abuts onto the ring-shaped gasket 744 of the Luer connector thereby providing a tight seal between the Luer connector and the patient interface.

Fig. 7B illustrates an alternate embodiment of the present invention using the same principles for the locking and sealing mechanisms to the one shown in Fig. 7A. However, in this embodiment, the Luer end 741 does not comprise a flexible second portion 743. Instead, the rigid portion 742 comprises at least one rigid ridge 745 that flexes due to gaps 749 formed in a surrounding region.

Reference is now made to Figs. 8A to 8C, which are side cross sectional views of a first end of a Luer connector, constructed and operative in accordance with further embodiments of the present invention.

Fig. 8A illustrates a Luer end 841 of a high flow Luer connector adapted to receive a corresponding end of a patient interface (not shown). The Luer end 841 may comprise a rigid portion 842 having a plurality of channels 850 defined on an inner surface. The channels 850 may be filled with a flexible material such as, for example, but not limited to, a flexible thermoset elastomer or thermoplastic suitable to form ridges 845 adapted to grip tabs provided on an outer surface of a patient interface fitting (not shown). Those skilled in the art will appreciate that the particular number of channels illustrated in Fig. 8A is not limiting and that any suitable number of channels may be defined in the inner surface of the rigid portion 842 so as to accommodate patient interface fittings of different dimensions. In such embodiment, the sealing mechanism is provided by the locking or retention mechanism (push-on attachment) formed by tabs of the patient interface fitting and the ridges 845. Figs. 8B and 8C illustrates a particular configuration of the embodiment shown in Fig. 8A. In such embodiment, only one channel 850 is provided and filled with a flexible material. A patient interface fitting 837 is shown in Fig. 8C with tabs 839 gripped by the ridges 845.

Although the connection between the second end of the Luer connector and the patient interface fitting was described using threads/tabs and complementary threads/ridges in relation to the description of Figs. 2 to 8C, it will be appreciated by those skilled in the art that any suitable retention and/or sealing mechanism may be provided so as to enable the Luer end of the Luer connector to lock and/or seal around the outer surface of the patient interface fitting.

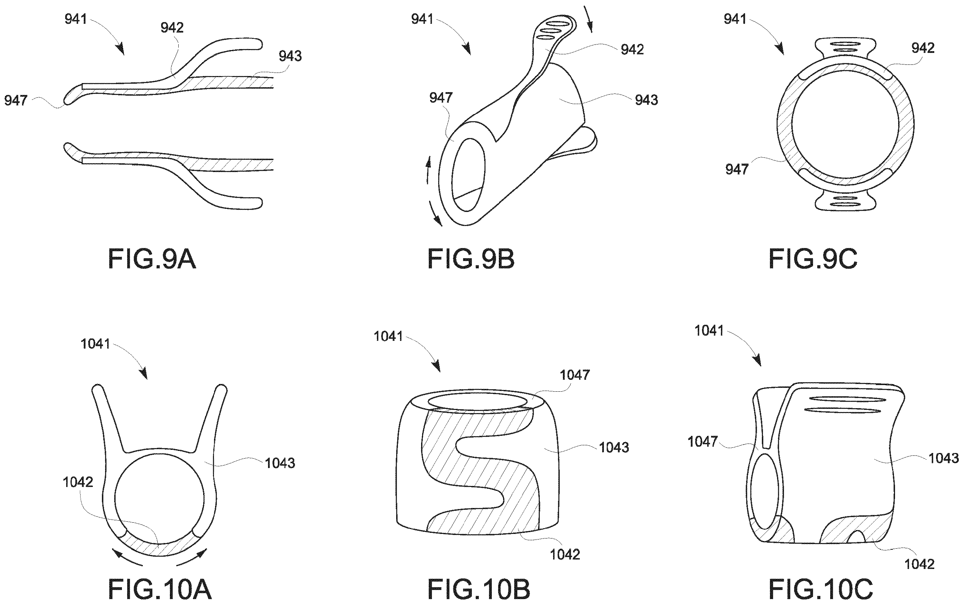

Reference is now made to Figs. 9A to 9C, which are different views of a first end of a Luer connector, constructed and operative in accordance with another embodiment of the present invention.

Figs. 9A-9C show several views of a Luer end 941 of a high flow Luer connector adapted to receive a corresponding end of a patient interface (not shown). The Luer end 941 comprises a first rigid portion 942 and a flexible second portion 943. The flexible second portion 943 may be made of any suitable flexible material such as, for example, but not limited to, a flexible thermoset elastomer or thermoplastic and comprises a distal end 947 that defines an opening adapted to receive the patient interface fitting (not shown). The rigid first portion 942 may be made of any suitable material such as, for example but not limited to, a rigid thermoset or thermoplastic material. The rigid first portion 942 may be attached, at one end, to an outer surface of the flexible second portion 943 close to the distal end 947 by any suitable means such as, for example, but not limited to, chemical or mechanical bonding. The rigid first portion 942 may also be shaped so as to form levers at a second end that are disposed along the gases flow path but spaced apart and away from the outer surface of the flexible second portion 943.

In this embodiment of the present invention, the diameter of an inner surface of the second flexible portion 943 is smaller than the diameter of an outer surface of the patient interface fitting. Therefore, to connect the patient interface fitting to the Luer end 941 of the Luer connector, the user may squeeze the levers so as to increase the size of the opening defined by the distal end 947 of the second flexible portion 943 and allow the patient interface fitting to be inserted. When the levers are released, the flexible second portion 943 tightens over the patient interface fitting, holding it in place and sealing onto it. Although Figs. 9A-9C show two diametrically opposite levers, those skilled in the art will appreciate that any suitable number and positions of levers may be provided as long as it enables a user to stretch the opening of the flexible second portion 943 and allow the insertion of the patient interface fitting.

Reference is now made to Figs. 10A to 10C, which are different views of a first end of a Luer connector, constructed and operative in accordance with a further embodiment of the present invention.

Figs. 10A-10C show several views of a Luer end 1041 of a high flow Luer connector adapted to receive a corresponding end of a patient interface (not shown). The Luer end 1041 comprises a first rigid portion 1042 and a flexible second portion 1043. The rigid first portion 942 may be made of any suitable material such as, for example but not limited to, a rigid thermoset or thermoplastic material. The flexible second portion 1043 may be made of any suitable flexible material such as, for example, but not limited to, a flexible thermoset elastomer or thermoplastic and disposed on a first surface of the Luer connector (e.g. on a bottom surface as shown in Figs. 10A-10C). The rigid first portion 1042 may be attached to the flexible second portion 1043 by any suitable means such as, for example, but not limited to, chemical or mechanical bonding. The rigid first portion 1042 may also be shaped so as to form levers that are disposed on a second surface of the Luer connector which is substantially opposite to the flexible second portion 1043. The levers may be adjacent to and extends radially and away from the gases flow path.

In this embodiment of the present invention, the diameter of an inner surface of the Luer connector at a distal end 1047 is smaller than the diameter of an outer surface of the patient interface fitting. Therefore, to connect the patient interface fitting to the Luer end 1041 of the Luer connector, the user may push the levers so as to increase the size of the opening defined at the distal end 1047 by deformation of the flexible second portion 1043 and allow the patient interface fitting to be inserted. When the levers are released, the Luer end 1041 of the Luer connector tightens over the patient interface fitting, holding it in place and sealing onto it. Although Figs. 10A-10C show two levers, those skilled in the art will appreciate that any suitable number and positions of levers may be provided as long as it enables a user to increase the size of the opening and allow the insertion of the patient interface fitting.

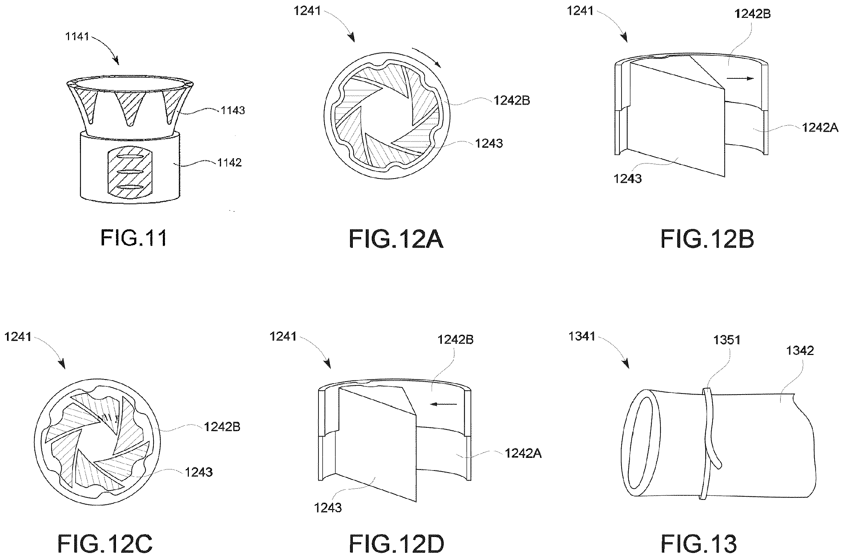

Reference is now made to Fig. 11, which is a side view of a first end of a Luer connector, constructed and operative in accordance with an embodiment of the present invention.

Fig. 11 illustrates a Luer end 1141 of a high flow connector adapted to receive a corresponding end of a patient interface (not shown). The Luer end 1141 comprises a rigid first portion 1142 and a second portion 1143. The second portion 1143 is substantially rigid but comprises a plurality of inserts made of a flexible material and disposed so as to form rigid finger-like sections. When the Luer end 1141 of the Luer connector is open, the rigid finger-like sections of the second portion 1143 are wide enough to fit over the patient interface fitting. In other words, when the Luer end 1141 is open, the inner diameter of the Luer connector is greater than an outer diameter of the patient interface fitting. The rigid first portion 1142 may be a sleeve adapted to slide over the second portion 1143 forcing the rigid finger-like sections together to close over an outer surface of the patient interface fitting and create a seal.

Reference is now made to Figs. 12A-D, which are views of a first end of a Luer connector, constructed and operative in accordance with a further embodiment of the present invention.

Figs. 12A-12D illustrate a Luer end 1241 of a high flow Luer connector adapted to receive a corresponding end of a patient interface (not shown). The Luer end 1241 comprises a rigid first portion comprising two rings 1242a and 1242b adapted to rotate relative to each other. Alternatively, a first one of these rings (e.g. ring 1242a) may be fixed and the second one (e.g. ring 1242b) may be adapted to rotate relative to the first one. The Luer end 1241 further comprises a flexible second portion 1243 having a plurality of flexible blades. Each blade is attached to one of the rings (e.g. ring 1242a in Figs. 12B and 12D) and comprises a convex section adapted to fit into a corresponding concave section positioned on the other one of the rings (e.g. ring 1242b in Figs. 12B and 12D). When the Luer end 1242 of the Luer connector is open, the concave sections of one of the ring are engaged with the convex sections of the blades and therefore, the inner diameter of the Luer connector is greater than an outer diameter of the patient interface fitting. When the patient interface fitting is inserted into the Luer connector, an operator may twist one the rings (e.g. ring 1242b) forcing the concave sections of the ring to be dislodged from the convex sections of the blades. As a result, the blades of the flexible portion rotate to close over an outer surface of the patient interface fitting and create a seal (Figs. 12B and 12D).

Reference is now made to Fig. 13, which is a side view of a first end of a Luer connector, constructed and operative in accordance of a further embodiment of the present invention.

Fig. 13 illustrates a Luer end 1341 of a high flow connector adapted to receive a corresponding end of a patient interface (not shown). The Luer end 1341 comprises a flexible portion 1042 that may be made of any suitable flexible material such as, for example, but not limited to, a flexible thermoset or thermoplastic material. The diameter of an inner surface of the Luer end 1341 is greater than the diameter of an outer surface of the patient interface fitting to be inserted. The Luer end 1341 may be sealed and secured to a patient interface fitting by a fastening element 1351. The fastening element 1351 may be any suitable element operative to seal and secure the Luer end 1341 to the patient interface such as, for example, but not limited to, a velcro fastening assembly, a releasable ratchet tie-strap assembly, a hook-and-loop fastening assembly, etc.

It will be appreciated by those skilled in the art that Figs. 2 to 8C illustrate different embodiments of the present invention in which the retention mechanism engages during attachment motion without requiring any actuation mechanism. The seal may be provided by the retention mechanism or by a separate mechanism. By contrast, it will be apparent to those skilled in the art that Figs. 9A to 13 illustrate further embodiments of the present invention in which the retention mechanism may be actuated by a user (e.g. physician, nurse, etc.) before, during or after attachment motion. Additionally, in such embodiments, the seal is provided by the retention mechanism.

Reference is now made to Figs. 14 to 17, which are side cross sectional views of a second end of a Luer connector, in accordance with embodiments of the present invention.

Fig. 14 illustrates a tubing end 1460 of a high flow Luer connector adapted to be coupled to a conduit end 1412 such as, for example, but not limited to, the end of the patient conduit 112 of Fig. 1. The tubing end 1460 may comprise a connector having barb 1462 and boss 1463 projections adapted to be coupled with outer and inner tubings of the conduit 1412 as shown in Fig. 14. The barb projection 1462 may be inserted within the inner tubing of the conduit 1412. The boss projection 1463 may be inserted within the outer tubing of the conduit 1412. Fig. 14 also shows the flexible body of the Luer lock connector and/or the flexible portion of the Luer end being overmoulded onto the connector. More specifically, the body and/or Luer end is overmoulded onto the barb projection 1463.

Fig. 15A illustrates a tubing end 1560 similar to the one shown in Fig. 14. However, in such embodiment, the barb and boss projections are integrated in a single connector 1562. The boss portion of the single connector 1562 may be offset toward the body and/or Luer end 1561 to expose the barb portion and the outer tubing of the conduit end 1512 may extend further toward the flexible body and/or Luer end 1561 than the inner tubing.

Fig. 15B illustrates a tubing end 1560 similar to the ones shown in Figs. 14 and 15A. The tubing end 1560 of the high flow Luer connector is adapted to be coupled to a conduit end 1512. The tubing end 1560 may comprise barb 1562 and boss 1563 portions adapted to be coupled with outer 1513 and inner 1514 tubings of the conduit 1512. The barb portion 1562 may be inserted within the inner tubing 1514 of the conduit 1512. The boss portion 1563 may be inserted within the outer tubing 1513 of the conduit 1512. By contrast to the embodiment depicted on Fig. 15A, the barb portion 1562 of Fig. 15B includes a tapered end while the boss portion 1563 is an annular projection instead of a step. The tapered end allows for easier insertion of the barb portion 1562 into the inner tubing 1514. The boss portion 1563 may also include a sloped or ramped surface 1564 to allow for easier insertion of the boss portion 1563 into the outer tubing 1513.

The conduit 1512 as shown in Fig. 15B has an inner tubing 1514, and an outer tubing 1513. The inner tubing 1514 provides for a lumen or gases pathway, to allow for the passage of gases along and through the tube. The inner tubing 1514 may pneumatically seal with the barb portion 1562 as described above and as shown in Fig 15B. The seal between the inner tubing 1514 and the barb portion 1562 may be by formed by one or more of: deformation of the inner tube around the barb portion 1562, or an adhesive, or an overmould. The outer tubing 1513 is located outward or external to the inner tubing 1514. The outer tubing 1513 may pneumatically seal with the boss portion 1563 as described above and as shown in Fig 15B. The seal between the outer tubing 1513 and the boss portion 1563 may be by formed by one or more of: deformation of the inner tube around the barb portion 1562, or an adhesive, or an overmould. In some embodiments, the barb portion 1562 may act as a stop or surface to engage with an end of the inner tubing 1514 to prevent over insertion of the barb portion 1562 within the inner tubing 1514. Similarly, in some embodiments a part of the connector may act as a stop for the outer tubing 1513 (for example a cuff at the end of the outer tubing 2213.)

The inner tubing 1514 and outer tubing 1513 may provide for a space therebetween. The space may define an insulation layer. The insulation layer may comprise an air gap to insulate the inner tubing 1514 with respect to the surrounding environment. The conduit 1512 may also include a heater wire 1515 configured to heat the gases in the conduit 1512. The heater wire 1515 may be located in the lumen of the inner tube, and/or located in or on a wall of the inner tube.

Fig. 16 illustrates a tubing end 1660 comprising a rigid cuff connector 1662 extending inside conduit 1612. The conduit end 1612 - either a single tubing or the inner and outer tubings of a dual-tubing conduit - may be bonded to the rigid cuff connector 1662 by the overmoulded body and/or Luer end 1661 of the Luer lock connector.

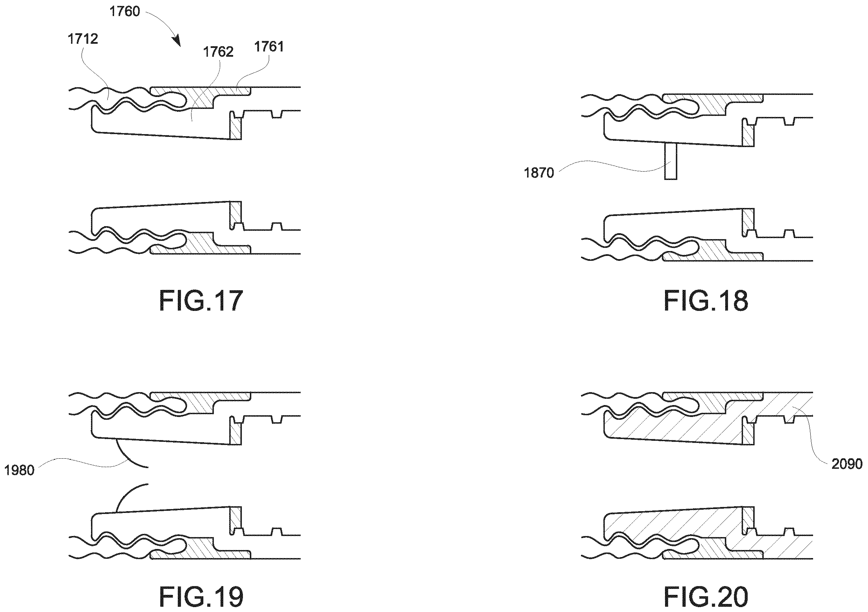

Fig. 17 illustrates a tubing end 1760 similar to the one shown in Fig. 16. However, in such embodiment, the overmoulded body and/or Luer end 1761 of the Luer lock connector flows into the inside of the rigid cuff connector 1762 through small gaps (not shown) thereby strengthening the bond between the connector 1762 and the body/Luer end 1761.

It will be appreciated by those skilled in the art that the body and/or Luer end 1461, 1561, 1661 and 1761 may be bonded with the rigid connectors 1462, 1562, 1662 and 1762 by any suitable means such as, for example, but not limited to, mechanical or chemical bonding.

Reference is now made to Figs. 18 to 20, which are cross sectional views of a second Luer end of a Luer connector, in accordance with other embodiments of the present invention.

Fig. 18 illustrates a tubing end of a high flow Luer connector that may comprise a sensor 1870. The sensor 1870 may be located in the gases flow path and within the Luer connector. The sensor 1870 may be configured to measure one or more operating parameter related to the gases flow such as, for example, but not limited to, a temperature, a pressure, humidity and a flow rate of the gases. Alternatively, a plurality of sensors may be provided and disposed in the gases flow path and within the Luer connector. The sensor 1870 may be further configured to transmit the measured data to the humidifier 104 of Fig. 1 for instance and/or to any other local or remote component of the insufflation system 100. The measured data may be transmitted by any suitable means such as, for example, but not limited to, a wire associated with the conduit 112 (e.g. inside the inner tubing, between the inner and outer tubings, on the outside of the outer tubing, or embedded within either the inner or outer tubings), in a flying lead, or wirelessly using RFID (Radio-Frequency Identification) or Wi-Fi technologies, etc. Non-limiting examples of how the data may be used include: using the measured temperature and/or humidity data in closed loop control of the humidifier; using the measured flow rate and/or pressure data to display the actual pressure drop from the gases source to the patient interface; using the measured flow rate and/or pressure data in closed loop control of the gases source if such control input is available, etc.

Fig. 19 illustrates a tubing end of a high flow Luer connector that may comprise a non-return valve 1980. The non-return or check valve 1980 may be located in the Luer connector to ensure that the gases cannot flow back toward the humidifier and/or gases source. The non-return valve 1980 may be, for example, a flutter valve which is thin and flexible enough to allow forward flow but collapse under backflow. Those skilled in the art will appreciate that any valve designed to minimize the resistance to flow may be used with the high flow Luer connectors described in the different embodiments of the present invention.

It will be further appreciated by those skilled in the art that the sensor 1870 and the valve 1980 may be disposed at any suitable location within the Luer connector and not merely in the tubing end as depicted in Figs. 18 and 19.

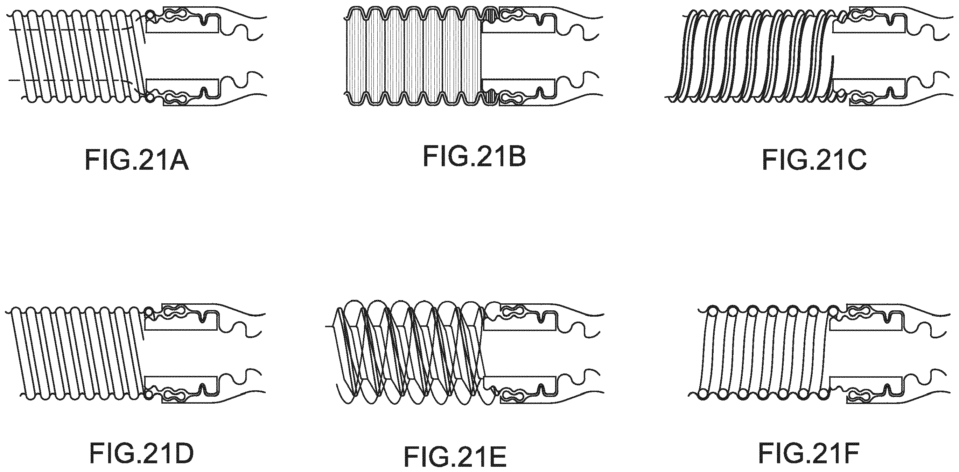

Fig. 20 illustrates a tubing end of a high flow Luer connector that comprises one or more portions 2090 made of a gas indicator material. The one or more portions 2090 may comprise a "litmus" type of material that changes color as indicator of the presence of a particular gases characteristic. A non-limiting example may include the use of a carbon dioxide indicator dye such as bromothymol blue as disclosed in U.S. Patent Application No. 2013/0220326 (Fisher & Paykel Limited). The one or more portions 2090 may comprise rigid portions of the Luer connector that may comprise or be impregnated with the dye, such that at least part of the dye is in contact with the gases flow and at least the same or another part is visible by an external user. It will be appreciated that other types of gases indicators or indicators for other gases characteristics (e.g. humidity level, temperature, etc.) may be used.
Reference is now made to Figs. 21A to 21F, which are side cross sectional views of a second end of a Luer connector connected to different tube arrangements, constructed and operative in accordance with embodiments of the present invention.

The tubing end of the high flow Luer connector may be attached to any dual-tubing conduit or any type of single tubing conduit, including the conduit disclosed in Figs. 21A to 21F. Non-limiting examples of conduit that may be attached to the tubing end of the Luer connector includes the conduit of: Fig. 21B having annular corrugations as disclosed in U.S. Patent Application No. 2013/0098360 (Fisher & Paykel Limited); Fig. 21C having helical crested corrugations; Fig. 21D having helical corrugations as disclosed in U.S. Patent Application No. 2013/0233318 (Fisher & Paykel Limited); Fig. 21E having an helical bead and bubbles as disclosed in PCT Patent Application WO 2015/142192 (Fisher & Paykel Limited); and Fig. 21F having an helical bead and a film as disclosed in PCT Patent Application WO 2016/048172 (Fisher & Paykel Limited).

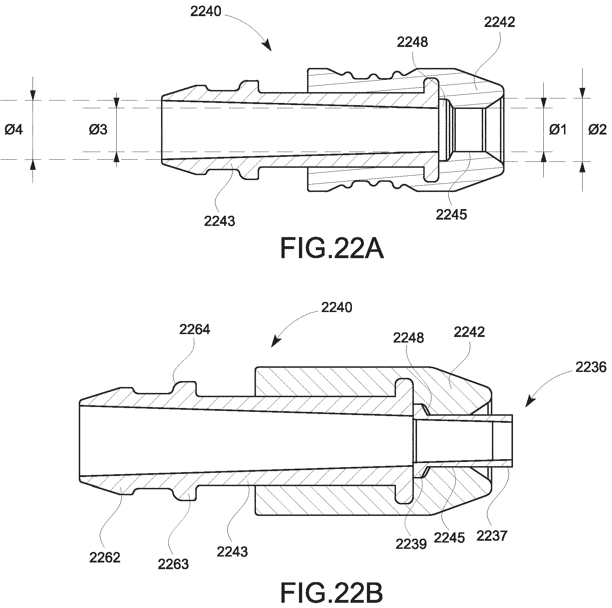

Reference is now made to Figs. 22A to 22D, which are different views of a Luer connector, constructed and operative with an embodiment of the present invention.

As it is apparent from the previous figures, the Luer connector 2240 of Figs. 22A-22D is a combination of the Luer end 541 of Fig. 51 (being for example a second portion) and the tubing end 1560 (being for example a first portion) of Fig. 15B. The Luer connector 2240 may comprise a body having an interior region defining a gases flow passageway allowing insufflation gases to flow through. The body may comprise a first portion 2243 including a first end which, in use, is configured to attach to the tubing of a patient conduit (e.g. the patient conduit 112 of Fig. 1). Further, the body may comprise a second portion 2242 including a second end, which, in use, is configured to removably connect to a fitting of a patient interface (e.g. the patient interface 136).

Fig. 22A is a cross-sectional view showing the interior construction of the Luer connector 2240. The second portion 2242 may be made of any suitable flexible material adapted to be overmoulded onto the first portion 2243. The second portion 2242 may comprise a ridge 2245 (e.g. long and tight neck portion) to seal around an outer surface of a patient interface fitting thereby creating a single seal between the second portion 2242 and the outer surface of the patient interface fitting. In other words, a seal is formed only between the second end of the second portion 2242 and the outer surface of the patient interface fitting. The second portion may further comprise a confined area 2248 adjacent the ridge 2245. The confined area 2248 is configured to receive and retain a flanged portion (e.g. tabs) located on a distal end of the patient interface fitting. The confined area 2248 may also force the flanged portion to abut against the Luer connector 2240, for example a surface, and/or a flange of the first portion 2243. By receiving and retaining the flanged portion of the patient interface connector in the confined area 2248, the risk of obstructing the gases flow path when the Luer connector is pulled/tugged is reduced. In some embodiments, the second portion 2242 may be overmoulded onto the first portion 2243 such that material is provided between the confined area 2248 (defined by the second portion 2242) and the first portion 2243. The material provided between the confined area 2248 (of the second portion 2242) and the first portion 2243 may define a ring-shaped gasket as described above. This latter arrangement creates a further seal (in addition to the one provided by the neck portion or ridge 2245) between the flanged portion of the patient interface fitting and the second portion 2242 of the Luer connector 2240. In addition, Fig. 22A shows a second portion 2242 with a tapered outer surface which does not comprise an external lip. This arrangement improves the rigidity of the Luer lock connector 2240 and reduces the risk of occlusion of the gases flow path, and/or reduces the risk of disconnection from the patient interface when forces are applied from the sides.

As explained hereinabove in relation to Figs. 5B and 22A, a patient interface fitting 2237 is adapted to be connected to the Luer connector 2240 as illustrated in Fig. 22B. At the second end, the second portion 2242 comprises a confined area 2248, a neck region or ridge 2245 (also referred hereinafter as an intermediate neck region), and a frustroconical opening - e.g. the opening has an inner diameter varying from a first diameter proximal to an intermediate neck region or ridge 2245 to a second diameter distal from the intermediate region 2245, the first diameter being less or smaller than the second diameter. The frustroconical opening is adapted to receive and guide the patient interface fitting 2237 during insertion of the patient interface 2236. Then, when the fitting 2237 is pushed further, the intermediate neck region or ridge 2245 is adapted to deform so to allow passage of the fitting 2237. Lastly, the confined area 2248 is adapted to receive, engage and retain a flanged end 2239 of the fitting 2237. To do so, the inner diameter of the confined area 2248 may be, in some embodiments, greater than the inner diameter of the intermediate neck region or ridge 2245 as illustrated on Figs. 5B-5I and Fig. 22A. When connected, the intermediate neck region 2245 conforms around and/or presses the outer surface of the patient interface fitting 2237 (e.g. a shaft portion of the patient interface fitting 2237) thereby providing a tight seal between the Luer connector 2240 and the patient interface 2236.

The Luer connector of Figs. 22A and 22B is a high flow Luer connector as the first 2243 and second 2242 portions are configured and/or dimensioned so as to provide sealing and retention features (described hereinabove) with less resistance to gases flow than traditional Luer connectors of the art. In certain embodiments, the second portion 2242 may be configured and/or dimensioned to have an inner diameter which is larger or the same as the inner diameter of the patient interface fitting 2237. For example, the smallest inner diameter of the second portion 2242 may be larger or the same size as the smallest inner diameter of the patient interface fitting 2237. In another example, the inner diameter of the intermediate neck region or ridge 2245 is larger or the same size as the smallest inner diameter of the patient interface fitting 2237. The second portion 2242 may be made of a flexible material so as to expand in cross-section during insertion and after connection of the patient interface fitting 2237 to the Luer connector 2240. Figs. 22A and 22B also show that the first 2243 and second 2242 portions have different inner diameters. Table 1 below provides non-limiting examples for these inner diameters.

**Table 1 - Examples of inner diameters for the Luer connector**

| Feature | | Dimension (mm) | Range (±) |
|---|---|---|---|
| Ø1 | Inner diameter of the intermediate neck region or ridge (second portion 2242) | 5.20 | 0.5 |
| Ø2 | Inner diameter of the confined area (second portion 2242) | 7.50 | 1 |
| Ø3 | Inner diameter of the first portion 2243 proximal to the conduit end | 5.00 | 1.75 |
| Ø4 | Inner diameter of the first portion 2243 distal to the conduit end | 7.20 | 3 |

Those skilled in the art will appreciate that the diameters provided in Table 1 are not limiting and that any suitable diameters for the inner sections of the first 2243 and second 2242 portions may be defined so as to provide a high flow Luer connector with a low resistance to gases flow. In certain embodiments, the inner diameter of the intermediate neck region or ridge 2245 can be in the range of 4.7 mm and 5.7 mm, and more particularly in the range of 4.8 mm and 5.7 mm. In addition, the inner diameter of the intermediate neck region or ridge 2245 can be a discrete dimension such as for example, but not limited to, 5 mm, 5.2 mm or 5.4 mm. In certain embodiments, the inner diameter of the first portion 2243 proximal to the conduit end can be in the range of 3.25 mm and 6.75 mm, and more particularly in the range of 3.9 mm and 6.75 mm. The material of the flexible second portion 2242 may also configured to have a particular hardness. In certain embodiments, the hardness of the flexible second portion 2242 can be in the range of 40 A and 90A, more particularly in the range of 50A to 80A, or of 60A to 70A. In one embodiment, the hardness of the flexible second portion 2242 can be a Shore hardness of 40 A. The hardness of the second portion 2242 may be chosen to allow for easy engagement of the patient interface fitting with the Luer connector, while also still providing for sufficient hardness to create and maintain a seal in high flow conditions, and to retain the patient interface fitting to prevent disconnection 2237.

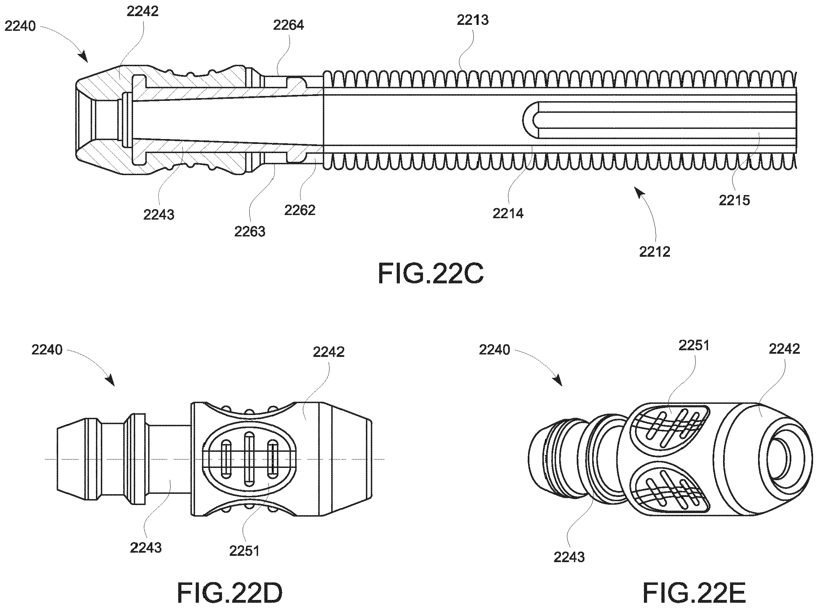

Fig. 22C illustrates the Luer connector 2240 of Figs. 22A and 22B being connected to a conduit 2212. The second portion 2243 may comprise barb 2262 and boss 2263 portions adapted to be coupled with outer 2213 and inner 2214 tubings of the conduit 2212. The barb portion 2262 (also shown in Fig. 22B) may be inserted within the inner tubing 2214 of the conduit 2212. The boss portion 2263 (also shown in Fig. 22B) may be inserted within the outer tubing 2213 of the conduit 2212. The barb portion 2262 may comprise a tapered end while the boss portion 2263 may comprise an annular projection. The tapered end allows for easier insertion of the barb portion 2262 into the inner tubing 2214. The boss portion 2263 may also include a sloped or ramped surface 2264 to allow for easier insertion of the boss portion 1563 into the outer tubing 2213.

The conduit 2212 as shown in Fig. 22C has an inner tubing 2214, and an outer tubing 2213. The inner tubing 2214 provides for a lumen or gases pathway, to allow for the passage of gases along and through the tube. The inner tubing 2214 may pneumatically seal with the barb portion 2262. The seal between the inner tubing 2214 and the barb portion 2262 may be by formed by one or more of: deformation of the inner tube around the barb portion 2262, or an adhesive, or an overmould. The outer tubing 2213 is located outward or external to the inner tubing 2214. The outer tubing 2213 may pneumatically seal with the boss portion 2263. The seal between the outer tubing 2213 and the boss portion 2263 may be by formed by one or more of: deformation of the inner tube around the barb portion 2262, or an adhesive, or an overmould. In some embodiments, the barb portion 2262 may act as a stop or surface to engage with an end of the inner tubing 2214 to prevent over insertion of the barb portion 2262 within the inner tubing 2214. Similarly, in some embodiments a part of the connector may act as a stop for the outer tubing 2213 (for example a cuff at the end of the outer tubing 2213).

The inner tubing 2214 and outer tubing 2213 may provide for a space therebetween. The space may define an insulation layer. The insulation layer may comprise an air gap to insulate the inner tubing 2214 with respect to the surrounding environment. The conduit 2212 may also include a heater wire 2215 configured to heat the gases in the conduit 2212. The heater wire 2215 may be located in the lumen of the inner tube, and/or located in or on a wall of the inner tube.

Figs. 22D and 22E illustrate external views of the Luer connector of Figs.22A-C. In Figs. 22D and 22E, the outer surfaces of the first 2243 and second 2242 portions of the Luer connector 2240 are visible as well as gripping means 2251 positioned on the outer surface of the second portion 2242. The gripping means 2251 are provided to ease manipulation and use of the Luer connector 2240 especially during connection of the patient interface and/or tubing of the patient conduit. The gripping means 2251 may comprise three finger grips positioned in four recesses formed on the outer surface of the second portion 2242. Those skilled in the art will appreciate that the gripping means depicted on Figs. 22D and 22E are provided as examples only and that any suitable number of finger grips and/or recesses may be provided. Similarly, it will be appreciated that any suitable gripping means may be provided on the outer surface of the second portion 2242 as long as it facilitates manipulation and use of the Luer connector 2240.

It will be further appreciated by those skilled in the art that the high flow Luer connector of the present invention may comprise any suitable combination of Luer ends as depicted in Figs. 2-13 and 22A-22E and tubing ends as depicted in Figs. 14-17 and 22A-22E so as to provide sealing and retention features with less resistance to gases flow than the traditional Luer connectors of the art.

There have been described and illustrated herein several embodiments of a high flow Luer connector. While particular embodiments of the invention have been described, it is not intended that the invention be limited thereto, as it is intended that the invention be as broad in scope as the art will allow and that the specification be read likewise. Thus, while particular types of Luer and tubing ends have been disclosed, it will be appreciated that any suitable combination of Luer and tubing ends may be used to provide a high flow connector. In addition, while particular types of materials, valves, sensors, gases indicator materials and conduits have been disclosed, it will be understood that other types can be used. It will therefore be appreciated by those skilled in the art that yet other modifications could be made to the provided invention without deviating from its spirit and scope as claimed.

It will be appreciated that there is thus disclosed:
According to the present invention there is provided a Luer lock connector for use in an insufflation system, said Luer lock connector comprising: a body comprising a first end, a second end and an interior region; said interior region defining a gases flow passageway allowing insufflation gases to flow through said body from said first end to said second end; and said body being configured to be coupled to a tubing arrangement at said first end and to a patient interface at said second end; wherein, said second end is configured to be coupled to a patient interface fitting of said patient interface, said second end being further configured to seal around an outer surface of said patient interface fitting when said Luer lock connector is coupled to said patient interface; and wherein, said second end forms a seal with said outer surface of said patient interface fitting when said second end and said patient interface fitting are coupled.

Preferably said seal between the second end and said outer surface of said patient interface fitting is the only seal between the patient interface and said second end.

Preferably said second end comprises an opening, and/or a neck region, and/or a confined area, optionally said neck portion is intermediate of said opening, and said confined area.

Preferably said opening comprises an inner diameter varying from a first diameter proximal to said neck region to a second diameter distal from said neck region, the first diameter being less than the second diameter.

Preferably said opening is adapted to receive and guide said patient interface fitting during insertion into said second end.

Preferably said neck region is adapted to deform to allow passage of said patient interface fitting.

Preferably said patient interface fitting comprises a flanged end portion and said neck region is adapted to deform to allow passage of said flanged end portion.

Preferably said confined area is adapted to receive and retain said patient interface fitting when said Luer lock connector and said patient interface fitting are coupled.

Preferably said patient interface fitting comprises a flanged end portion and said confined area is adapted to receive and retain said flanged end portion when said Luer lock connector and said patient interface fitting are coupled.

Preferably said neck portion conforms around an outer surface of said patient interface fitting to form a seal when said second end and said patient interface fitting are coupled.

Preferably said seal is formed only between said neck region and said outer surface of said patient interface fitting.

Preferably said patient interface fitting comprises a shaft portion and said neck portion conforms around an outer surface of said shaft portion to form said seal when said second end and said patient interface fitting are coupled, optionally said seal is provided along a length of a shaft of the patient interface connector.

Preferably said second end comprises an inner diameter which is larger or the same as an inner diameter of said patient interface fitting.

According to the present invention there is provided a Luer lock connector for use in an insufflation system, said Luer lock connector comprising: a body comprising a first end, a second end and an interior region; said interior region defining a gases flow passageway allowing insufflation gases to flow through said body from said first end to said second end; and said body being configured to be coupled to a tubing arrangement at said first end and to a patient interface at said second end; wherein, said second end is configured to be coupled to a patient interface fitting of said patient interface, said second end being further configured to seal around an outer surface of said patient interface fitting when said Luer lock connector is coupled to said patient interface; and wherein, said second end forms a seal with said outer surface of said patient interface fitting when said second end and said patient interface fitting are coupled; and wherein, said second end comprises a neck region adapted to deform to allow passage of said patient interface fitting.

Preferably said deformation of said neck region is configured to form said seal with said outer surface of said patient interface fitting.

Preferably said second end further comprises a ring-shaped seal being disposed in use between an end of said patient interface fitting and an annular flange on an inner surface of said second end, optionally said ring-shaped seal is provided at least in part by a surface of the second end.

Preferably said second end further comprises an opening, and/or a confined area, optionally said neck portion is intermediate of said opening, and said confined area.

Preferably said opening comprises an inner diameter varying from a first diameter proximal to said neck region to a second diameter distal from said neck region, the first diameter being less than the second diameter.

Preferably said opening is adapted to receive and guide said patient interface fitting during insertion into said second end.

Preferably said neck region is adapted to deform to allow passage of said patient interface fitting.

Preferably said patient interface fitting comprises a flanged end portion and said neck region is adapted to deform to allow passage of said flanged end portion.

Preferably said confined area is adapted to receive and retain said patient interface fitting when said Luer lock connector and said patient interface fitting are coupled.

Preferably said patient interface fitting comprises a flanged end portion and said confined area is adapted to receive and retain said flanged end portion when said Luer lock connector and said patient interface fitting are coupled.

Preferably said neck portion conforms around an outer surface of said patient interface fitting to form said seal when said second end and said patient interface fitting are coupled.

Preferably said seal is formed only between said neck region and said outer surface of said patient interface fitting.

Preferably said patient interface fitting comprises a shaft portion and said neck portion conforms around an outer surface of said shaft portion to form said seal when said second end and said patient interface fitting are coupled, optionally said seal is provided along a length of a shaft of the patient interface connector.

Preferably said second end comprises an inner diameter which is larger or the same as an inner diameter of said patient interface fitting.

Preferably said patient interface fitting is configured to be inserted into said second end of said Luer lock connector.

Preferably said second end comprises a flexible material and one or more ridges on an inner surface.

Preferably said second end is configured to be more flexible or softer than said first end.

Preferably said one or more ridges are configured to be coupled to a mating structure arranged on said outer surface of said patient interface fitting.

Preferably said one or more ridges and said mating structure form said seal when said second end and said patient interface fitting are coupled.

Preferably said second end further comprises a distal end having an inner diameter that is less than an outer diameter of said patient interface fitting, and wherein said distal end presses onto said outer surface of said patient interface fitting to form a seal when said second end and said patient interface fitting are coupled.

Preferably said second end comprises a first portion made of a rigid material and a second portion made of a flexible material.

Preferably said second portion is overmoulded over said first portion and said first portion comprises one or more gaps allowing said second portion to form one or more ridges on an inner surface of said first portion when said second portion is overmoulded over said first portion.

Preferably said second end comprises a flexible material and an inner diameter of said second end deforms and/or expands in cross section when said second end and said patient interface fitting are coupled, said deformation providing a sealing force forming said seal between the second end and said patient interface fitting.

Preferably an inner diameter of an inner surface of said second end is less than an outer diameter of said patient interface fitting.

Preferably said first end is configured to be coupled to a dual-tubing conduit.

Preferably said first end comprises boss and barb connectors, said boss connector comprising a projection configured to be inserted within an outer tubing of said dual tubing conduit, and said barb connector comprising a projection configured to be inserted within an inner tubing of said dual-tubing conduit.

Preferably said first end comprises a connector having boss and barb portions, said barb portion being longitudinally offset from said boss portion and comprising a projection configured to be inserted within an inner conduit of said dual-tubing conduit, and an outer tubing of said dual-tubing conduit extends further toward said first end than said inner tubing.

According to the present invention there is provided a Luer lock connector for use in an insufflation system, said Luer lock connector comprising: a body comprising a first end, a second end and an interior region; said interior region defining a gases flow passageway allowing insufflation gases to flow through said body from said first end to said second end; and said body being configured to be coupled to a tubing arrangement at said first end and to a patient interface at said second end; wherein, said second end is configured to be coupled to a patient interface fitting of said patient interface, said second end being further configured to create a single seal around an outer surface of said patient interface fitting when said Luer lock connector is coupled to said patient interface; and wherein, said second end forms said single seal with said outer surface of said patient interface fitting when said second end and said patient interface fitting are coupled.

The Luer lock connector of original PCT claim 41, wherein the Luer lock is defined by any one of original PCT claims 2-13 or 15-40.

According to the present invention there is provided a connector assembly comprising: a patient interface connector configured to be coupled to or with a patient interface, said patient interface connector comprising an outer surface having a flanged end portion and a shaft portion; and a Luer lock connector comprising first and second portions, said first portion being configured to be coupled to or with a tubing arrangement, and said second portion being configured to be coupled to or with said patient interface connector; wherein, said second portion of said Luer lock connector comprises a confined area and a neck region, and wherein said flanged end portion of said patient interface connector is configured to be received and retained in said confined area and said neck region is configured to seal around an outer surface of said shaft portion when said Luer lock connector is coupled to said patient interface connector.

The connector assembly of original PCT claim 43, wherein the Luer lock connector is defined by any one of original PCT claims 1-42.

## Claims

1. A Luer connector for coupling to a patient interface fitting, the Luer connector comprising:
a body comprising:
a first portion;
a second portion; and
an interior region defining a gases passageway through the first portion and the second portion;
wherein:
the second portion comprises a confined region configured to receive and retain the patient interface fitting; and
material is provided between the confined region and the first portion.

2. The Luer connector of Claim 1, wherein the second portion is configured to create a seal around an outer surface of the patient interface fitting.

3. The Luer connector of Claim 1 or 2, wherein the second portion comprises an opening configured to receive and guide the patient interface fitting.

4. The Luer connector of Claim 3, wherein the second portion comprises a ridge configured to deform to allow passage of the patient interface fitting, wherein the ridge is located between the opening and the confined region,
wherein optionally:
the opening comprises a first diameter proximal to the ridge and a second diameter distal from the ridge, wherein the first diameter is less than the second diameter; and/or
an inner diameter of the confined region is greater than an inner diameter of the ridge.

5. The Luer connector of any one of the preceding claims, wherein the second portion is overmoulded onto the first portion.

6. The Luer connector of any one of the preceding Claims, wherein the second portion comprises an external lip on an outer surface of the second portion.

7. The Luer connector of any one of the preceding Claims, wherein at least part of the second portion is provided between the confined region and the first portion,
wherein optionally:
the at least part of the second portion provided between the confined region and the first portion comprises a tapered transition.

8. The Luer connector of any one of the preceding Claims, wherein the first portion comprises a first material and the second portion comprises a second material, the second material being softer or more flexible than the first material.

9. The Luer connector of any one of the preceding Claims, wherein an outer portion of the second portion comprises slits configured to facilitate bending of one end of the second portion.

10. The Luer connector of any one of the preceding Claims, wherein the confined region is at least partially defined by the ridge.

11. The Luer connector of any one of the preceding Claims, wherein the second portion is configured to create a single seal around an outer surface of the patient interface fitting.

12. The Luer connector of any one of Claims 4 to 10, wherein the second portion comprises a second ridge configured to deform to allow passage of the patient interface fitting, wherein the second ridge is located between the opening and the confined region.

13. A connector assembly comprising:
the Luer connector of any one of the preceding Claims; and
a patient interface fitting,
wherein optionally:
an inner diameter of an end of the Luer connector is less than an outer diameter of the patient interface fitting.

14. The connector assembly of Claim 13, wherein the patient interface fitting comprises a distal end comprising at least one tab or flanged portion, wherein the confined region is configured to receive and retain the at least one tab or flanged portion,
wherein optionally:
the at least one tab or flanged portion is configured to seal with the confined region.

15. A kit for an insufflation system for delivering gases to a patient, the kit comprising:
the Luer connector of any one of Claims 1 to 12; and
at least one of:
a patient conduit configured to be coupled to one end of the Luer connector,
a humidifying chamber,
a filter,
a supply tube configured to be coupled between a gases source and an inlet of the humidifying chamber, or
a patient interface.
